# EUROPEAN PATENT APPLICATION

(11) **EP 1 156 118 A1**
(43) Date of publication of application: **21.11.2001**
(21) Application number: 00905319.0
(22) Date of filing: 24.02.2000
(51) Int. Cl.: C12N 15/86, A61K 48/00, A61P 35/00

(54) **VIRUS VECTOR**

(30) Priority: 24.02.1999 JP 9326399
(71) Applicant: JURIDICAL FOUNDATION JAPANESE FOUNDATION FOR CANCER RESEARCH, Tokyo 170-0012 (JP)
(72) Inventor: HAMADA, Hirofumi, Sapporo-shi, Hokkaido 064-0811 (JP)
(74) Representative: VOSSIUS & PARTNER
(86) International application number: JP0001069
(87) International publication number: WO0050618

(57) **Abstract**

There have been desired a virus vector useful for treatment and diagnosis of tumors such as malignant melanoma which is resistant to conventional therapeutic methods and poor in prognosis, and a diagnostic method and a therapeutic method of tumors using the virus vector.

The present invention provides a virus vector comprising a virus structural protein fused with a ligand which specifically binds to a melanocyte-stimulating hormone (MSH) receptor, and a diagnostic agent and therapeutic agent for a tumor using the vector.

## Description

### TECHNICAL FIELD

The present invention relates to a virus vector comprising a virus structural protein fused with a ligand which specifically binds to a melanocyte-stimulating hormone (MSH) receptor, and to a diagnostic agent and therapeutic agent for a tumor using the vector.

### BACKGROUND ART

Malignant melanoma which accompanies metastasis is resistant to conventional therapeutic methods, such as radiotherapy, chemotherapy and the like, and therefore its prognosis is extremely poor. Accordingly, development of a new effective therapeutic method has been strongly desired. On the other hand, many clinical studies on a cancer treating method using gene transfer via a virus vector or the like (gene therapy for cancers) have been started in recent years with high expectations.

However, an effective gene transfer method to achieve sufficient therapeutic effect for malignant melanoma has not been established yet. For example, gene transfer efficiency for malignant melanoma cells is not sufficient with the conventional virus vectors, such as retrovirus, adenovirus, adenovirus-associated virus (AAV) and the like.

The present inventor has examined gene transfer efficiency of the current adenovirus vectors for malignant melanoma cells, and reported that sufficient gene transfer efficiency could not be obtained even if a virus having relatively high MOI (multiplicity of infection) was used (Yoshida *et al., Hum Gene Ther., 9*(17): 2503-2515, 1998 (hereinafter referred to as Yoshida *et al*., 1998)). Among the results shown in the report, the gene transfer efficiency obtained at MOI 100 is merely about 50% for A375 human malignant melanoma cells, 80% for RPMI 7951 malignant melanoma cells and 50% for WM 115 malignant melanoma cells, so that administration of a larger amount of adenovirus is necessary for obtaining a gene transfer efficiency close to 100%.

The current virus vectors have disadvantages in that not only is the efficiency of gene transfer simply low but also, the gene is introduced non-selectively into normal cells around melanoma cells. That is, they have low gene transfer efficiency as a vector for treatment of malignant melanoma, and the selectivity for malignant melanoma is also poor.

Attempts for modifying the host-range by introducing a mutated amino acid sequence into the C-terminal or HI loop moiety of the fiber protein of adenovirus have been reported by the groups of Wickham *et al.* at GenVec, USA and Curiel *et al.* at Alabama University (Wickham *et al., J. Virol., 71*: 8221 (1997); Wickham *et al., Gene Ther., 2*: 750 (1995); Wickham *et al., Nat. Biotechnology, 14: 1570* (1996); Curiel *et al., Hum. Gene Ther, 3*: 147 (1992); Dimitriev *et al., J. Virol., 72:* 9706 (1998); WO 94/10323; WO 96/07734).

It has been reported that an MSH receptor is present in human malignant melanoma and MSH binds thereto (Siegrist *et al., Cancer Res., 49*: 6352 (1989)). Accordingly, the inventor expects that a virus vector prepared by fusing MSH to the outer coat protein of a virus will become a vector which can introduce a gene efficiently into malignant melanoma. However, there is no report concerning successfully preparing a vector containing an MSH ligand, nor is there any report which proved a possibility of gene therapy using the MSH receptor as a target. Examples of virus vectors other than adenovirus include those in which a cell growth factor (erythropoietin) (Kasahara *et al., Science, 266:* 1373 (1994)) or a single chain antibody (Jiang *et al., J. Virol., 72(12):* 10148 (1998)) was inserted and fused to the envelope protein of retrovirus for targeting, or those in which erythropoietin is fused with the C glycoprotein of herpes simplex virus (Laquerre *et al., J. Virol., 72*(12): 9683 (1998)). According to the report of Jiang et *al.,* Her2neu belonging to the EGF (epidermal growth factor) receptor family, CD34 which is considered to be specific for bone marrow stem cells and transferrin receptor were examined as a target antigen for the single chain antibody. Also, regarding reports on chimeric virus proteins into which an RGD motif capable of binding to integrin is artificially inserted, there were the reports on adenovirus by the group of Wickham (Wickham *et al., J. Virol.,* 71: 8221 (1997)) and the group of Curiel (Dimitriev et *al., J. Virol., 72:* 9706 (1998)), and reports of the core protein of type B hepatitis virus (Chambers *et al., J. Virol., 70*: 4805 (1996); Sharma *et al., Virology, 239:* 150 (1997)) and bacteriophage Fd protein (Koivunen *et al., J. Biol. Chem., 268:* 20205 (1993); Koivunen *et al., J. Cell Biol., 124:* 373 (1994)) as on viruses other than adenovirus. However, in viruses other than adenovirus, there is no report concerning a virus vector containing an MSH ligand. Nor is there any report which proved a possibility of gene therapy targeted at an MSH receptor.

### DISCLOSURE OF THE INVENTION

For the purpose of developing gene therapy of malignant melanoma, the current virus vectors exhibit poor efficiency and poor selectivity of gene transfer. Accordingly, there is a demand for a means capable of transferring a gene efficiently and selectively to malignant melanoma which is resistant to the conventional therapy and has extremely poor prognosis.

An object of the present invention is to provide a virus vector comprising a virus structural protein fused with a ligand which specifically binds to an MSH receptor, said virus vector being useful for the treatment and diagnosis of tumors including malignant melanoma, and an application method of the virus vector.

The present inventors found that the problems of gene transfer methods using conventional virus vectors of being low in efficiency and having poor selectivity for malignant melanoma can be solved by the use of a virus vector comprising a virus structural protein fused with a ligand which specifically binds to the MSH receptor, and thus the present invention has been accomplished. In addition, the action mechanism of the said virus vector can also be applied to a vector using other virus whose structural protein can be fused with a ligand which specifically binds to an MSH receptor as well as fiber protein of adenovirus.

The gene transfer having high efficiency and excellent selectivity has been achieved by the use of such a virus vector as a gene transfer vector for tumors expressing the MSH receptor such as malignant melanoma and the like.

Accordingly, the present invention provides a virus vector having high efficiency and high selectivity for tumors expressing the MSH receptor such as malignant melanoma and the like, as well as a recombinant virus vector which is prepared based on the vector. By selecting the gene to be introduced, such a recombinant virus vector is useful as a vector for diagnosis and treatment which can be applied to a diagnostic agent for specifically identifying tumor cells expressing the MSH receptor such as malignant melanoma and the like, a cancer treating agent which specifically kills the tumor cells, or an immunotherapeutic agent for a cancer which specifically increases antigenicity of the tumor.

Conventional adenovirus vectors are mainly recombinants using human adenovirus type 5 or 2, and have a gene transfer efficiency for malignant melanoma of around MOI 100 as the amount of virus from which 50% transfer (ED₅₀) can be obtained (Yoshida *et al*., 1998). That is, the transfer efficiency for malignant melanoma is not so high. On the other hand, since the gene transfer efficiency for normal cells is similar or higher, a high efficiency of gene transfer specific for malignant melanoma cannot be expected. By the use of the virus vector of the present invention, 1) markedly higher transfer efficiency for tumors expressing the MSH receptor such as malignant melanoma and the like than in conventional methods is obtained and 2) the transfer efficiency for normal cells around the tumor is similar to or lower than that of the conventional vectors, so that gene transfer having high specificity for tumors expressing the MSH receptor such as malignant melanoma and the like can be obtained. Accordingly, 1) when a high expression level is necessary, higher gene expression in targeted tumor cells than that by the conventional methods can be obtained even when the same amount of virus is used as the conventional methods, so that superior therapeutic effects can be obtained as a result. Also, 2) when using a gene from which sufficient therapeutic effects can be obtained at a relatively low expression level, dose of the virus can be reduced by the use of the vector of the present invention. As a result, The present invention permits to alleviation of undesirable side effects accompanied by the virus administration (allergy reaction, injury of normal cells around a tumor and the like).

In addition, when the present invention is integrally combined with a proliferating recombinant virus such as adenovirus having E1A or the like, infection efficiency and proliferation and re-infection of the virus in tumor tissues after the infection are synergistically increased, so that such provides a remarkably effective therapeutic method.

Thus, the present invention has high utility for the treatment of tumors expressing the MSH receptor such as malignant melanoma and the like.

Preparation of a mutant adenovirus in which a ligand other than MSH is inserted into the C-terminal of the fiber protein or the like has already been reported. However, there are many cases in which the adenovirus cannot be produced due to the insertion of a ligand. Even if the virus is produced, there are many cases in which the product does not have the expected affinity for the receptor (see Wickham *et al., J. Virol., 71*(11): 8221-8229 (1997)). Thus, even if a vector is prepared using a fusion protein derived from a known ligand for targeting a known receptor, the possibility of obtaining a useful virus vector is completely unpredictable until it is practically prepared and allowed to infect. For example, Wickham *et al.* have prepared vectors by inserting a motif sequence (TRSDITWDQLWWDLMKTS) which binds to E-selectin or a motif sequence (TSAA(SIKVAV)₂) which binds to a laminin receptor into the C-terminal of adenovirus fiber protein, but a recombinant adenovirus was not produced. Also, it has been reported that when a vector and a recombinant adenovirus thereof were prepared by inserting a sequence TS(GRGDTF)₃SS containing a RGD motif which binds to αν-integrin or a motif sequence TS(GYIGSR)₃SS which binds to a laminin receptor in the same manner, no specific binding to the expected receptors was found (Wickham *et al., J. Virol., 71*(11): 8221-8229 (1997)).

In contrast to these conventional techniques, the virus vector of the present invention provides results which are far superior to the generally expected results for malignant melanoma.

Using the facts that the MSH receptor is expressed in many melanoma cells (Siegrist *et al., Cancer Res*., 49, 6352 (1989)) and that MSH as a ligand specifically binds to the MSH receptor with high affinity, the present inventor has completed a vector which can perform gene transfer by efficiently infecting malignant melanoma cells. The vector of the present invention is effective for not only malignant melanoma but also other tumors expressing the MSH receptor.

The present invention relates to the following (1) to (26):
(1) A virus vector comprising a virus structural protein fused with a ligand which specifically binds to an MSH receptor.
(2) The virus vector according to (1), wherein the virus structural protein is fused with a ligand which specifically binds to the MSH receptor via a linker.
(3) The virus vector according to (2), wherein the linker is an oligopeptide.
(4) The virus vector according to (3), wherein the linker has the amino acid sequence represented by any one of SEQ ID NOs:25, 27, 29 and 31.
(5) The virus vector according to any one of (1) to (4), wherein the virus structural protein is a protein which constructs the outer surface of the virus.
(6) The virus vector according to any one of (1) to (5), wherein the ligand is a ligand selected from the group consisting of α-MSH, β-MSH, γ-MSH and derivatives of any one thereof.
(7) The virus vector according to any one of (1) to (6), wherein the virus is selected from viruses belonging to any one of the group consisting of the family *Adenoviridae,* the family *Retroviridae,* the family *Parvoviridae,* the family *Herpesviridae,* the family *Poxviridae,* the family *Papovaviridae*, the family *Hepadnaviridae,* the family *Togaviridae,* the family *Flaviviridae,* the family *Coronaviridae,* the family *Rhabdoviridae,* the family *Paramyxoviridae,* the family *Orthomyxoviridae,* the family *Bunyaviridae,* the family *Arenaviridae* and the family *Reoviridae.*
(8) The virus vector according to any one of (1) to (6), wherein the virus is a human adenovirus.
(9) The virus vector according to any one of (1) to (8), wherein the virus contains an exogenous gene.
(10) The virus vector according to (9), wherein the gene is a gene encoding an enzyme capable of converting a nontoxic prodrug into a drug having a cytotoxicity.
(11) The virus vector according to (10), wherein the gene is a gene encoding a herpes simplex virus thymine kinase (HSV-tk) or a cytosine deaminase (CD).
(12) The virus vector according to (9), wherein the gene is a gene encoding a molecule having a cytotoxic activity directly or indirectly.
(13) The virus vector according to (12), wherein the gene is a gene encoding a cytokine, a cell growth factor or a cell growth inhibiting factor.
(14) The virus vector according to (12), wherein the gene is a tumor repressor gene, a cell cycle regulator gene or a cell death regulator gene.
(15) The virus vector according to (9), wherein the exogenous gene is a wild type or mutant gene of adenovirus E1A or E1B or a part of the gene.
(16) A medicament comprising the virus vector according to any one of (1) to (15).
(17) An antitumor agent comprising the virus vector according to any one of (1) to (15).
(18) The antitumor agent according to (17), wherein the tumor is malignant melanoma.
(19) A diagnostic agent for a tumor, comprising the virus vector according to any one of (1) to (15).
(20) The diagnostic agent according to (19), wherein the tumor is malignant melanoma.
(21) A linker comprising the amino acid sequence represented by any one of SEQ ID NOs:25, 27, 29 and 31.
(22) A DNA encoding the linker according to (21).
(23) A DNA comprising the nucleotide sequence represented by any one of SEQ ID NOs:24, 26, 28 and 30.
(24) A protein comprising the amino acid sequence represented by any one of SEQ ID NOs:32 to 39.
(25) A DNA encoding the virus vector according to (24).
(26) A DNA comprising the nucleotide sequence represented by any one of SEQ ID NOs:7, 13, 17, 18, 20, 21, 22 and 23.

Examples of the virus vector of the present invention include viruses belonging to any one of the groups consisting of the family *Adenoviridae,* the family *Retroviridae*, the family *Parvoviridae,* the family *Herpesviridae,* the family *Poxviridae,* the family *Papovaviridae,* the family *Hepadnaviridae,* the family *Togaviridae,* the family *Flaviviridae,* the family *Coronaviridae,* the family *Rhabdoviridae,* the family *Paramyxoviridae,* the family *Orthomyxoviridae,* the family *Bunyaviridae,* the family *Arenaviridae* and the family *Reoviridae,* and vectors derived from these viruses, adenovirus dodecahedron vector (Fender *et al., Nature Biotech., 15:* 52-56 (1997)), vectors in which a virus is combined with liposome (for example, Sendai virus with a liposome vector *etc*.) and the like. Among these, a human adenovirus is preferably used.

The virus vector of the present invention can be prepared by replacing a coding region of a DNA encoding a virus structural protein to encode a fusion protein of the virus structural protein and an MSH ligand, using general recombinant DNA techniques (see *Molecular cloning: A laboratory manual,* 2nd ed., edited by Sambrook *et al.,* Cold Spring Harbor Laboratory Press, New York, 1989, etc.). The recombinant viruses or those complexed with liposome and the like can be prepared in accordance with a known method. Examples of the known method include methods described in the following references:

Wolff ed., *Gene therapeutics:* Methods and applications of direct gene transfer, Birkhaeuser, Boston, 1994; Kaplitt and Loewy eds., *Viral vectors:* Gene therapy and neuroscience applications, Academic Press, San Diego, 1995; Liu *et al.* eds., *DNA vaccines:* A new era in vaccinology, Annals of the New York Academy of Sciences, vol. 772, The New York Academy of Sciences, New York, 1995; Gluzman and Hughes eds., *Viral vectors:* Current communications in molecular biology, Cold Spring Harbor Laboratory, New York, 1988; and *Methods in cell biology,* vol. 43, Protein expression in animal cells, Academic Press, San Diego, 1994.

Examples of the virus structural protein used in the present invention include a protein which constructs the outer surface of a virus. Examples of the protein which constructs the outer surface of a virus include G protein of VSV (vesicular stomatitis virus), envelope protein of retrovirus (env), capsid protein of adenovirus (hexon, penton base, fiber), hemagglutinin of influenza virus, surface glycoprotein of paramyxovirus and the like, but any virus protein involved in the adsorption to the surface of a host cell such as a cancer cell or the like or the interaction with a specific receptor can be used in the present invention.

Examples of the ligand capable of specifically binding to the MSH receptor used in the present invention include α-MSH, β-MSH, γ-MSH and the like. Also, it is possible to artificially prepare those which have stronger affinity for the MSH receptor than the natural MSH, by preparing their mutants (such as those which are screened from their derivatives and random peptides). All of these MSH and MSH-like ligands are included in the ligand to be used in the present invention.

In the following descriptions, the ligand which specifically binds to the MSH receptor is simply referred to as MSH, and all ligands having strong affinity for the MSH receptor can be used in the same manner in the present invention.

Although the MSH and a virus protein can be fused directly, they also can desirably be fused via a linker peptide. As the linker peptide, an oligopeptide having a length of about 1 to 100 residues can be used. Regarding the sequence of the oligopeptide, any peptide which can join a virus protein and MSH while keeping the MSH function can be used in the present invention. In this regard, the linker peptide may have a specified peptide sequence already reported in a literature or an unreported peptide sequence. Also, it is immaterial to the present invention if it is attached position being the C-terminal, inside or at the N-terminal position of MSH, also immaterial are its length, sequence and the like.

The position where MSH is fused to a virus structural protein may be any position of the C-terminal, inside and N-terminal of the virus protein. For example, the N-terminal of MSH can be fused with the C-terminal of a virus structural protein such as an adenovirus fiber protein via an oligopeptide having the amino acid sequence represented by any one of SEQ ID NOs:25, 27, 29 and 31. The linker peptide having the amino acid sequence represented by any one of SEQ ID NOs:25, 27, 29 and 31 and DNA encoding the peptide are also included in the present invention.

Examples of the virus structural protein fused with MSH via a linker peptide of the present invention include a protein having the amino acid sequence represented by any one of SEQ ID NOs:32 to 39 and the like. DNAs encoding the protein are also included in the present invention. Specific examples include DNAs having the nucleotide sequence represented by any one of SEQ ID NOs:7, 13 and 17 to 23 and the like.

The gene can be introduced efficiently into a target cell by inserting an exogenous gene into the virus vector of the present invention which is also included in the present invention. For example, a target cell such as a cancer cell or the like can be killed efficiently and selectively by inserting, as an exogenous gene, a gene which encodes a molecule having a cytotoxic activity on a target cell directly or indirectly. Examples of such a gene include those encoding a cytokine, a cell growth factor, a cell growth inhibiting factor and the like, also a tumor repressor gene, a cell cycle regulator gene, a cell death regulator gene and the like.

Additionally, a target cell can be made into sensitive for a prodrug by inserting, as an exogenous gene, a gene encoding an enzyme capable of converting a nontoxic prodrug into a drug having a cytotoxicity, such as herpes simplex virus thymine kinase (HSV-tk), cytosine deaminase (CD) or the like. For example, when a gene encoding HSV-tk is inserted, the target cell can be made into sensitive for ganciclovir or aciclovir. When a gene encoding CD is inserted, 5-fluorocytosine which is nontoxic in the target cell can be converted into 5-fluorouracil which is a drug having a cytotoxicity.

Also, examples of the exogenous gene include a wild type or mutant gene of adenovirus E1A or E1B, or it may be a gene containing a part of the gene.

The virus vector of the present invention can be used as a medicament, for example, a therapeutic drug for a tumor expressing MSH such as malignant melanoma or the like, particularly a therapeutic drug for malignant melanoma.

The medicament containing the virus vector of the present invention can be administered by the vector alone as the therapeutic drug, but it is generally preferred to provide the vector as a pharmaceutical preparation produced by an optional method well known in the technical field of manufacturing pharmacy, by mixing it with one or more pharmaceutically acceptable carriers. Preferably, a sterile solution prepared by dissolving it in water or an aqueous carrier such as an aqueous solution or the like of sodium chloride, glycine, glucose, human albumin is used. Also, pharmaceutically acceptable additives 'such as a buffering agent, a tonicity agent and the like for use in resembling the pharmaceutical preparation solution to physiological conditions, e.g., sodium acetate, sodium chloride, sodium lactate, potassium chloride, sodium citrate and the like can be added. Of course, it is possible to store the preparation by freeze-drying for later use by dissolving it in an appropriate solvent when used.

It is preferred to use a route of administration which is most effective in treatments, and a parenteral route, for example, an administration route such as subcutaneous, intramuscular, intravenous, airway or the like is generally used.

The therapeutic drug containing the vector of the present invention can be administered by the vector alone as the therapeutic drug, but it is generally preferred to provide it as a pharmaceutical preparation produced by an optional method well known in the technical field of manufacturing pharmacy, by mixing it with one or more pharmaceutically acceptable carriers.

It is preferred to use a route of administration which is most effective in treatments, and its examples include oral administration or parenteral administration such as buccal, airway, rectal, subcutaneous, intramuscular, intravenous or the like. The dosage form includes sprays, capsules, tablets, granules, syrups, emulsions, suppositories, injections, ointments, tapes and the like.

Examples of the pharmaceutical preparation suitable for oral administration include emulsions, syrups, capsules, tablets, powders, granules and the like. For example, liquid preparations such as emulsions and syrups can be produced using, as additives, water, saccharides such as sucrose, sorbitol, fructose, *etc*.; glycols such as polyethylene glycol, propylene glycol, *etc*.; oils such as sesame oil, olive oil, soybean oil, *etc.;* antiseptics such as p-hydroxybenzoic acid esters, *etc*.; flavors such as strawberry flavor, peppermint flavor, *etc*.; and the like. Capsules, tablets, powders, granules and the like can be produced using, as additives, fillers such as lactose, glucose, sucrose, mannitol, *etc*.; disintegrating agents such as starch, sodium alginate, *etc*.; lubricants such as magnesium stearate, talc, *etc*.; binders such as polyvinyl alcohol, hydroxypropylcellulose, gelatin, *etc*.; surfactants such as fatty acid ester, *etc*.; plasticizers such as glycerol, *etc*.; and the like.

Examples of the pharmaceutical preparation suitable for parenteral administration include injections, suppositories, sprays and the like. For example, injections are prepared using a carrier such as a salt solution, a glucose solution or a mixture thereof. Suppositories are prepared using a carrier such as cacao butter or a hydrogenated fat or carboxylic acid. Also, sprays are prepared using the vector as such or using a carrier or the like which does not stimulate the buccal or airway mucous membrane of the patient and can facilitate absorption of the vector by dispersing it into fine particles. Examples of the carrier include lactose, glycerol and the like. Depending on the properties of the vector and the carrier, it is possible to produce pharmaceutical preparations such as aerosols, dry powders and the like. In addition, the components exemplified as additive agents for oral preparations can also be added to these parenteral preparations.

Although the dose or frequency of administration varies depending on the intended therapeutic effect, administration method, treating period, age, body weight, kind of the virus vector and the like, it is usually from 10³ to 10¹⁵ as the virus vector per administration per adult.

The virus vector of the present invention can be used as diagnostic drugs, e.g., as a diagnostic drug for tumors expressing MSH such as malignant melanoma and the like, particularly as a diagnostic drug for malignant melanoma. For example, tumors expressing MSH such as malignant melanoma and the like can be specifically detected by inserting a gene to be used as a marker into the virus vector of the present invention.

### BRIEF DESCRIPTION OF THE DPAWINGS

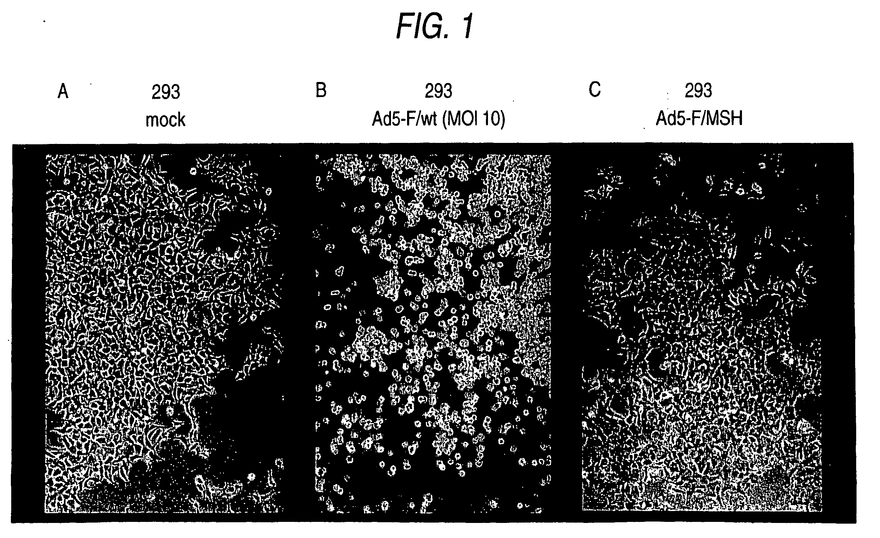

Fig. 1 is a morphological view of cells 4 days after culturing the 293 cells by infecting them with a wild type adenovirus Ad5dlX-F/wt and an F/MSH mutant adenovirus Ad5-F/MSH. A, B and C show results of infections with control mock, wild type adenovirus Ad5dlX-F/wt, and F/MSH mutant adenovirus Ad5-F/MSH, respectively.

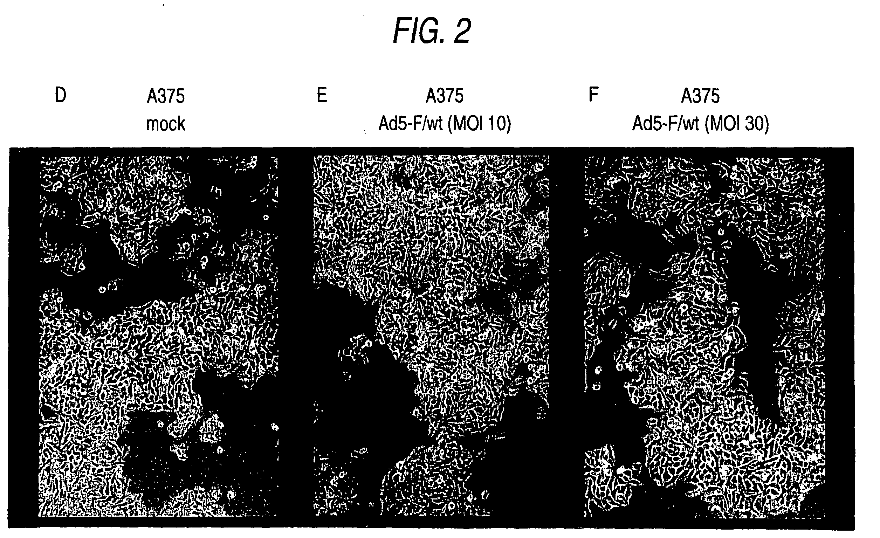

Fig. 2 is a morphological view of cells 4 days after culturing the A375 cells by infecting them with the wild type adenovirus Ad5dlX-F/wt. D, E and F show results of infection with control mock, wild type adenovirus Ad5dlX-F/wt at MOI 10, and wild type adenovirus Ad5dlX-F/wt at MOI 30, respectively.

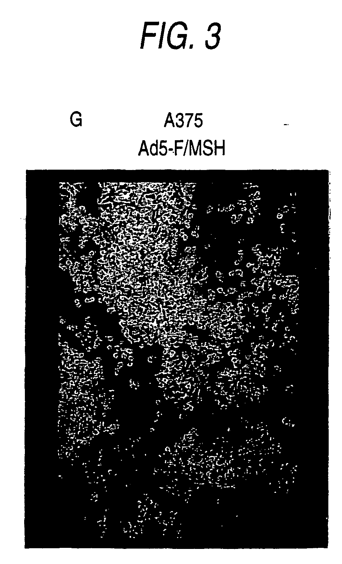

Fig. 3 is a morphological view of cells 4 days after culturing the A375 cells by infecting them with the F/MSH mutant adenovirus Ad5-F/MSH.

### BEST MODE FOR CARRYING OUT THE INVENTION

The present invention is specifically explained below using human adenovirus type 5 (Ad5) and the fiber of Ad5 as the virus vector and the virus structural protein to be fused with MSH, respectively, although the present invention is not limited to these examples.

### Example 1

### Preparation of human adenovirus type 5 having mutation of MSH-fused fiber (F/MSH) (hereinafter referred to as "Ad5-F/MSH"):

### a) Preparation of plasmid encoding F/MSH mutant:

A nucleotide sequence encoding a linker consisting of 11 amino acids and a human α-MSH consisting of 13 amino acids joined at the 3'-terminal of a coding region of the wild type fiber (SEQ ID NO:1) was synthesized by polymerase chain reaction (PCR) using a synthetic oligonucleotide No. 924 (126 mer, SEQ ID NO:2) as a template and No. 933 (SEQ ID NO:3) and No. 934 (SEQ ID NO:4) as primers.

This PCR product was digested with *Eco*RI and cloned into the *Eco*RI site of pBluescript SKII+ (manufactured by Stratagene) to thereby obtain pSKII+nbMSH (Yoshida *et al.,* 1998). Next, a *Hind*III/*Xho*I fragment of pSKII+nbH (Yoshida *et al.,* 1998) and a *Xho*I/*Mun*I fragment of pSKII+nbMSH were cloned into the *Hind*III/*Mun*I site of pSKII+[X-K] (Yoshida *et al.,* 1998) to thereby obtain plasmid pSKII+[X-K]nbMSH. Next, a *Nhe*I/*Kpn*I fragment (2.1 kbp) of pSKII+[X-K]nbMSH was subcloned into the *Nhe*I/*Kpn*I site of pSKII+6.7Rnp (Yoshida *et al.,* 1998) to thereby obtain pSKII+6.7R-MSH. An *Eco*RI/*Pac*I fragment was cut out from pSKII+6.7R-MSH and cloned into the *Eco*RI/*Pac*I of pTR (Yoshida *et al*., 1998) to thereby obtain pTR-MSH. A predicted C-terminal amino acid sequence of the F/MSH mutant fiber is shown below. Each number in the sequence indicates the position of the amino acid residue when counted by defining the N-terminal of the adenovirus type 5 (Ad5) fiber as 1.
S₅₇₁SYTFSYIAQE₅₈₁PSASASASAPG₅₅₂SYSMEHFRWGKPV₆₀₅

The amino acid sequence of the original Ad5 fiber is up to 581, the amino acid sequence of the linker is 11 residues from 582 to 592, and the amino acid sequence of human α-MSH is 13 residues from 593 to 605.

*Escherichia coli* DH5α/pTR-MSH containing pTR-MSH has been deposited on February 22, 1999, in National Institute of Bioscience and Human Technology, Agency of Industrial Science and Technology (1-3, Higashi 1-chome, Tsukuba, Ibaraki, Japan 305-8566), as FERM BP-6656.

### b) Preparation of F/MSH mutant recombinant adenovirus

The recombinant adenovirus having F/MSH was prepared in accordance with a known method (Yoshida *et al.,* 1998). That is, a genomic DNA-terminal protein complex (hereinafter referred to as "DNA-TPC") was isolated from adenovirus Ad5d1X (Miyake *et al., Proc. Natl. Acad. Sci. U.S.A., 93*: 1320-1324 (1996)), digested with *Eco*RI and *Ase*I and then co-transfected into the 293 cells together with PacI-digested plasmid DNA of pTR-MSH. Essentially the same method described as the F/K20 mutant preparation method by Yoshida *et al.,* 1998 was used. However, when the co-transfection was carried out in accordance with a known method (Miyake *et al., Proc. Natl. Acad. Sci. U.S.A., 93*: 1320-1324 (1996)), a plaque was not obtained even though the experiment was repeated (Yoshida et *al.,* 1998). Since the virus titer of the obtained Adv-F/MSH mutant was extremely low in this example, it was considered that it was difficult to isolate the virus by using the known virus plaque formation method. Accordingly, the present inventors have changed the inoculum size of the DNA-transfected 293 cells in a 96 well plate to 30% of the conventional method, lowered the concentration of fetal bovine serum (FBS) in the culture to 5% which was the half of the conventional 10%, and continued culturing for 3 weeks while optionally adding the culture medium in 50 µl per well 4, 8 and 15 days after the transfection to thereby at last isolate plaques of two clones as a whole. These viruses were amplified by infecting the 293 cells and A375 human malignant melanoma cells and then their biological activities were examined. The titer of the thus obtained virus solution of Ad5-MSH was below the detection limit (10⁵ pfu/ml or less) by the usual plaque assay method using the 293 cells.

### c) Investigation of cytotoxicity induced by infection of the 293 cells and A375 human malignant melanoma cells with F/MSH fiber mutant adenovirus

The 293 cells or A375 cells was spread onto a 6 well plate, followed by control mock infection, infection with a wild type (wt) adenovirus Ad5d1X-F/wt and infection with an F/MSH mutant adenovirus (Ad5-F/MSH) on the next day. Then, the morphology of the cells after 96 hours was observed under a phase contrast microscope. The control 293 cells are shown in Fig. 1A, and the 293 cells infected with Ad5d1X adenovirus of F/wt and F/MSH are shown in B and C, respectively. Almost 100% of the F/wt-infected 293 cells shown in B died and floated in round shapes by 4 days culturing. On the other hand, the F/MSH-infected 293 cells in C showed almost the same morphology as the control (A) causing almost no damage.

Fig. 2D shows the morphology of the control A375 cells, and those of the A375 cells infected with Ad5d1X adenovirus of the wild type fiber (F/wt) at MOI 10 and 30 in E and F, respectively. Contrary to the result of the 293 cells, sufficient cytotoxicity was not obtained in the F/wt-infected A375 cells by 4 days culturing (E and F in Fig. 2). On the other hand, Fig. 3G shows the morphology of the A375 cells infected with Ad5d1X adenovirus having F/MSH mutant fiber (Ad5-F/MSH). It can be seen that markedly strong cytotoxicity is obtained by 4 days culturing.

Based on the above results, it was shown that the F/MSH fiber mutant adenovirus is useful as a gene transfer vector having higher efficiency and also having more excellent selectivity for the A375 human malignant melanoma cells than the 293 cells.

### Example 2

### Preparation of improved human adenovirus type 5 having MSH-fused mutant fiber (Ad5-F/asMSHa):

Although Example 1 achieved significant effects of F/MSH mutant adenovirus on malignant melanoma, namely high efficiency of gene transfer and strong cytotoxic effect, the titer of the obtained virus solution was low (10⁵ pfu/ml or less). Accordingly, the inventor considered that it is necessary to improve the titer and obtained an MSH-fused fiber mutant adenovirus having a practically and sufficiently high titer of 10⁷ to 10⁸ pfu/ml or more using a fiber mutant adenovirus preparation method described below.

### a) Preparation of DNA fragment encoding F/asMSHa fiber mutant

A region encoding the α-MSH and a poly(A) signal region of the fiber were synthesized by PCR using newly synthesized oligonucleotides No. 1061 (SEQ ID NO:5) and No. 1092 (SEQ ID NO:6) as primers and the pSKII+6.7R-MSH in Example 1 as the template.

The PCR product was digested with *Bam*HI/*Eco*RI and cloned into the *Bam*HI/*Eco*RI site of pNEB193 (manufactured by NEB) to confirm its nucleotide sequence.

### b) Preparation of cosmid pWE6.7R-F/asMSHa

The cosmid pWE15 (GenBank accession, M99569) was purchased from Clontech (Palo Alto, CA, USA). The *Sac*II site of pSKII+6.7R-K20 (described on page 2506 of Yoshida *et al., Hum. Gene Ther., 9*: 2503-2515 (1998)) was blunt-ended with T4 DNA polymerase, and a newly synthesized phosphorylated *Bst*BI linker (pdGCTTCGAAGC) was inserted into the site. From this product, an *Eco*RI/*Bst*BI fragment containing Ad5 adenovirus genome was cut out and cloned into the *Eco*RI/*Cla*I site of pWE15 to thereby obtain pWE6.7R-F/K20. From the pWE6.7R-F/K20, pWE6.7R-F/asMSHa was obtained by replacing a *Bam*HI/*Kpn*I fragment containing a sequence encoding the K20 mutation with a *Bam*HI/*Kpn*I fragment of the No. 1061-No. 1092 PCR product described in a). In this connection, the nucleotide sequence of a fiber-encoding region (Ad5-F/asMSHa. seq) and the encoded amino acid sequence are shown in SEQ ID NO:7.

### c) Preparation of cosmid pWEAxKM-F/asMSHa

A *Bam*HI digestion fragment (1,264 bp) containing kanamycin resistance gene was cut out from plasmid pUC-4K purchased from Pharmacia, blunt-ended with T4 DNA polymerase and cloned into the *Swa*I site of pAx-cw (Miyake *et al., Proc. Natl. Acad. Sci. U.S.A., 93:* 1320-1324 (1996)) to thereby obtain pAxKM. By cloning an *Eco*RI fragment (about 25,773 bp) containing the Ad5 genome into the *Eco*RI site of the pWE6.7R-F/asMSHa described in b) and selecting a product in which the Ad5 genome is connected in the correct direction, the cosmid pWEAxKM-F/asMSHa having a total length of about 40,702 bp was obtained.

### d) Preparation of F/asMSHa mutant recombinant adenovirus

The DNA-TPC of adenovirus Ad5d1X was digested with *Eco*RI and *Ase*I and the pWEAxKM-F/asMSHa was digested with *Cla*I and *Pac*I, and they were co-transfected into the 293 cells. A large number of plaques could be isolated by a known cell culturing method without using the specific culturing method described in Example 1. It was confirmed from the result of virus genome analysis that it was a mutant of Ad5d1X having the expected F/asMSHa mutation. In order to distinguish from the virus described in Example 1, this mutant was named Ad5-F/asMSHa. A desirably high titer of 1.10×10⁸ pfu/ml for practical use was obtained from the stock solution of the Ad5-F/asMSHa virus.

### Example 3

### Preparation of F/asMSHa fiber mutant recombinant adenovirus AxCAZ3-F/asMSHa which expresses reporter lacZ gene:

In order to show the preparation of an F/asMSHa fiber mutant adenovirus in which an expression cassette of various exogenous genes were introduced into the E1A region, the inventor constructed a recombinant adenovirus which expresses *E. coli* lacZ gene as the reporter.

About 4,889 bp of an *Ase*I fragment (blunt-ended) containing lacZ was cut out from pCAZ2 described in Yoshida *et al.*, 1998, and cloned into the *Bgl*II/*Sal*I (blunt-ended) site of pCI plasmid purchased from Promega (Madison, WI, USA) to thereby obtain pCAZ3. A *Bgl*II/*Bam*HI fragment (blunt-ended) of about 5,153 bp was cut out from the pCAZ3 and cloned into the *Swa*I site of cosmid pAx-cw (Miyake et *al.*, 1996) to thereby obtain pAxCAZ3. Using this cosmid DNA and DNA-TPC of Ad5d1X, a recombinant adenovirus of wild type fiber (F/wt), AxCAZ3-F/wt, was obtained. The DNA-TPC was prepared from the AxCAZ3-F/wt and digested with *Eco*RI and *Ase*I, the WEAxKM-F/asMSHa cosmid obtained in Example 2 was digested with *Cla*I and *Pac*I, and they were co-transfected into the 293 cells. A large number of plaques could be isolated by a known culturing method, and it was confirmed from the result of virus genome analysis that it was a recombinant adenovirus having the expected F/asMSHa mutation and also having a reporter lacZ expression cassette in the E1A region. This recombinant virus was named AxCAZ3-F/asMSHa.

### Example 4

### Preparation of F/asMSHa mutant adenovirus using host cell which highly expresses MSH receptor (hereinafter referred to as "MSHR"):

### a) Preparation of retrovirus vector expressing MSHR

cDNA fragments encoding an N-terminal half and C-terminal half of MSHR were obtained by amplification (RT-PCR) using cDNA obtained from crude RNA of the human melanoma A375 cells as the template, and primers No. 1037 (SEQ ID NO:8) and No. 1040 (SEQ ID NO:11) as primers for the N-terminal half and No. 1038 (SEQ ID NO:9) and No. 1039 (SEQ ID NO:10) as primers for the C-terminal half. Each of the DNA fragments was digested with *Eco*RI/*Kpn*I and cloned respectively into the *Eco*RI/*Kpn*I site of pBluescript II SK+ and then the nucleotide sequences were confirmed. From these plasmids, the fragment encoding the N-terminal half was cut out with *Eco*RI/*Kpn*I, and the C-terminal half with *Kpn*I/*Not*I, and they were cloned into the *EcoR*I/*Not*I site of plasmid pRx-bsr for retrovirus preparation (Shinoura et *al., Human Gene Ther., 9*: 1983-1993 (1998)) by three part ligation to thereby obtain a plasmid pRxhMSHR. Using this plasmid, an MSH-expressing retrovirus producer cell ψCRIP/MSHR was established using the method described in a reference (H. Hamada *et al., Retrovirus Vector,* edited by The Japan Society of Gene Therapy: Handbook of Research and Development of Gene Therapy, Chapter 3, Transfer Techniques, in press, NTS, 1999).

### b) Preparation of 293 host cell-derived cell line which highly expresses MSHR

A 293/MSHR cell line which highly expresses MSHR was obtained by infecting the 293 cells with retrovirus in the culture supernatant of ψCRIP/MSHR.

### c) Amplification of F/asMSHa mutant adenovirus using 293/MSHR

When the titer of F/asMSHa mutant adenovirus was measured by plaque assay using the 293/MSHR cells instead of the 293 cells usually used as the host for adenovirus preparation, 3 to 10 times higher apparent titers were obtained than the titer values obtained by the 293 cells. This was believed to be due to the increased infection efficiency and plaque formation ratio of F/asMSHa mutant adenovirus caused by use of the 293/MSHR cells in comparison with use of the 293 cells. That is, it is considered that a virus solution having a higher titer can be prepared by use of the 293/MSHR cells as the host than use of the 293 cells.

### Example 5

### Preparation of β-MSH-fused fiber mutant recombinant adenovirus:

In Examples 1 to 4, examples of the preparation of human α-MSH-fused fiber mutant recombinant adenovirus were shown. A possibility of preparing a fiber mutant virus useful for the treatment of malignant melanoma cells was further examined on ligands other than α-MSH, using β-MSH as an example of the ligands.

### a) Preparation of plasmid DNA encoding β-MSH-fused fiber protein

A β-MSH-encoding primer No. 1075 for PCR was newly prepared (SEQ ID NO:12). PCR was carried out using No. 1075 and No. 1092 (described in Example 2) as primers and pSKII+6.7Rnp as the template, the thus obtained PCR product was digested with *Bam*HI and *Eco*RI and cloned into the *Bam*HI/*Eco*RI site of pNEB193, and then the nucleotide sequence was confirmed. A β-MSH-encoding DNA fragment was cut out with *Bam*HI/*Kpn*I and cloned into the *Bam*HI/*Kpn*I site of pWE6.7R-F/asMSHa obtained in Example 2 to thereby obtain pWE6.7R-F/asMSHb. By further cloning an *Eco*RI fragment (about 25 kbp) of pAxKM into *Eco*RI site of pWE6.7R-F/asMSHb in the sense direction, pWEAxKM-F/asMSHb cosmid DNA was obtained.

### b) Preparation of β-MSH-fused fiber mutant adenovirus

By co-transfecting DNA-TPC of Ad5dlX and DNA of pWEAxKM-F/asMSHb into the 293 cells in the same manner as in Example 2, a β-MSH-fused fiber mutant adenovirus Ad5-F/asMSHb was prepared. A large number of plaques were obtained, and it was also confirmed based on the results of the analysis of virus genome that the intended F/asMSHb mutant virus was obtained.

Also, a recombinant adenovirus AxCAZ3-F/asMSHb having an *E. coli* lacZ reporter gene expression cassette and also having a β-MSH-fused fiber mutant was prepared by co-transfecting DNA-TPC of AxCAZ3-F/wt and DNA of pWEAxKM-F/asMSHb into the 293 cells in the same manner as in Example 3. DNA sequence of the F/asMSHb mutant fiber is shown in SEQ ID NO:13.

### Example 6

### Preparation of MSH-fused fiber mutant adenovirus having GS linker:

In Examples 1 to 5, examples were shown on the preparation of a mutant adenovirus having a structure in which an amino acid sequence of α-MSH or β-MSH ligand was joined to the C-terminal of the fiber protein of adenovirus via an AS linker (PSASASASAPG, SEQ ID NO:25) (in the AS linker for β-MSH ligand, a serine residue is further added to the C-terminal). Regarding a linker other than the AS linker, a possibility of preparing a fiber mutant virus useful for the treatment of malignant melanoma cells was also examined using a GS linker (GSGSGSGSGSG, SEQ ID NO:27; for the β-MSH ligand, a serine residue is further added to the C-terminal) as an example.

### a) Preparation of plasmid DNA encoding GS linker

A GS linker-encoding primer No. 1060 (61 mer, SEQ ID NO:14) was newly synthesized. In addition, in order to facilitate the PCR, a primer No. 1098 (41 mer, SEQ ID NO:15) which is shorter than the No. 1060 and has a slightly different codon usage was newly synthesized. The PCR was carried out using pSKII+6.7R-K20 as the template and also using No. 931 (described in Yoshida *et al.,* 1998; SEQ ID NO:16) and No. 1060, and the PCR was further carried out using the PCR product as the template and No. 931 and No. 1098.

The PCR product was digested with *Hind*III/*Bam*HI and cloned into pNEB193, and then the nucleotide sequence was confirmed. Next, *Xho*I/*Bam*HI DNA fragments containing AS linkers of pWE6.7R-F/asMSHa and pWE6.7R-F/asMSHb were replaced by *Xho*I/*Bam*HI DNA fragment containing GS linker to thereby obtain pWE6.7R-F/gsMSHa and pWE6.7R-F/gsMSHb, respectively. By cloning an EcoRI fragment (about 25 kbp) of pAxKM into the *Eco*RI site of each cosmid DNA in the sense direction, pWEAxKM-F/gsMSHa and pWEAxKM-F/gsMSHb were obtained, respectively.

### b) Preparation of MSH-fused fiber mutant adenovirus having GS linker

Four MSH-fused fiber mutant adenoviruses having GS linker were established by co-transfecting the cosmids prepared in a) and the DNA-TPC of Ad5d1X or AxCAZ3-F/wt. That is, they were AdS-F/gsMSHa, Ad5-F/gsMSHb, AxCAZ3-F/gsMSHa and AxCAZ3-F/gsMSHb. It was confirmed based on the result of the analysis of virus genome that they are intended mutant adenovirus. Nucleotide sequences encoding the fibers of F/gsMSHa and F/gsMSHb and the encoded amino acid sequences are shown in SEQ ID NOs:17 and 18, respectively.

### Example 7

### Preparation of MSH-fused fiber mutant adenovirus having K21 linker:

In Examples 1 to 6, examples were shown on the preparation of a mutant adenovirus having a relatively short linker sequence of about 11 to 12 amino acids such as the AS linker or GS linker, but a possibility of preparing a fiber mutant virus useful for the treatment of malignant melanoma cells was further examined regarding linkers having longer amino acid sequence, using asK21 linker (SEQ ID NO:29) and gsK21 linker (SEQ ID NO:31) in which an amino acid sequence of 25 residues was added to the AS linker (11 amino acid) or GS linker (11 amino acid) (37 amino acids in total) as the examples.

### a) Preparation of plasmid DNA encoding asK21 linker or gsK21 linker

A K21 linker-encoding primer No. 1089 (128 mer, SEQ ID NO:19) was newly synthesized. The PCR was carried out using No. 1089 and No. 1092 as primers and pSKII+6.7Rnp as the template, the PCR product was cloned into the *Eco*RI site, and then the nucleotide sequence was confirmed. The DNA fragment from *Bgl*II site to *Bam*HI site as a region encoding the K21 linker was inserted into the *Bam*HI site of pWE6.7R-F/asMSHa, pWE6.7R-F/gsMSHa, pWE6.7R-F/asMSHb and pWE6.7R-F/gsMSHb to thereby obtain cosmid DNAs of pWE6.7R-F/asK21MSHa, pWE6.7R-F/gsK21MSHa, pWE6.7R-F/asK21MSHb and pWE6.7R-F/gsK21MSHb, respectively. An *Eco*RI fragment (about 25 kbp) of pAxKM was cloned into the *Eco*RI site of these cosmids encoding the fiber containing K21 linkers in the sense direction to thereby obtain cosmid DNAs of pWEAxKM-F/asK21MSHa, pWEAxKM-F/gsK21MSHa, PWEAxKM-F/asK21MSHb and pWEAxKM-F/gsK21MSHb, respectively.

### b) Preparation of MSH-fused fiber mutant adenovirus having asK21 linker or gsK21 linker

Adenovirus Ad5-F/asK21MSHa, Ad5-F/gsK21MSHa, Ad5-F/asK21MSHb and Ad5-F/gsK21MSHb were established by co-transfecting the cosmid DNA prepared in a) and the DNA-TPC of Ad5d1X, respectively. The DNA sequences of regions encoding the fibers of Ad5-F/asK21MSHa, Ad5-F/gsK21MSHa, Ad5-F/asK21MSHb and Ad5-F/gsK21MSHb and the encoded amino acid sequences are shown in SEQ ID NOs:20, 21, 22 and 23, respectively. In the same manner, adenovirus AxCAZ3-F/asK21MSHa, AxCAZ3-F/gsK21MSHa, AxCAZ3-F/asK21MSHb and AxCAZ3-F/gsK21MSHb were established by co-transfecting the DNA-TPC of AxCAZ3-F/wt and the cosmid DNAs, respectively. Based on the result of the virus genome analysis, it was confirmed that these 8 adenoviruses are the intended fiber mutant adenoviruses.

### INDUSTRIAL APPLICABILITY

The present invention can provide a virus vector comprising a virus structural protein fused with a ligand which specifically binds to an MSH receptor, and a diagnostic drug and therapeutic drug for a tumor expressing the MSH receptor such as malignant melanoma and the like, using the vector.

### SEQUENCE LISTING FREE TEXT

SEQ ID NO:1: DNA coding a part of adenovirus fiber type 5, AS linker peptide and α-MSH
SEQ ID NO:2: Synthetic DNA No. 924 used as template for PCR amplification of DNA sequence No. 1
SEQ ID NO:3: Synthetic DNA No. 933 used as sense primer for PCR amplification of DNA sequence No. 1
SEQ ID NO:4: Synthetic DNA No. 934 used as antisense primer for PCR amplification of DNA sequence No. 1
SEQ ID NO:5: Synthetic DNA No. 1061 used as sense primer for PCR amplification of DNA coding α-MSH and adenovirus fiber poly A signal
SEQ ID NO:6: Synthetic DNA No. 1092 used as antisense primer for PCR amplification of DNA coding α-MSH and adenovirus fiber poly A signal
SEQ ID NO:7: DNA coding a modified fiber protein of pWE6.7R-F/asMSHa
SEQ ID NO:8: Synthetic DNA No. 1037 used as sense primer for PCR amplification of DNA coding human MSH receptor residue 1-154
SEQ ID NO:9: Synthetic DNA No. 1038 used as antisense primer for PCR amplification of DNA coding human MSH receptor residue 150-317
SEQ ID NO:10: Synthetic DNA No. 1039 used as sense primer for PCR amplification of DNA coding human MSH receptor residue 150-317
SEQ ID NO:11: Synthetic DNA No. 1040 used as antisense primer for PCR amplification of DNA coding human MSH receptor residue 1-154
SEQ ID NO:12: Synthetic DNA No. 1075 used as sense primer for PCR amplification of DNA coding β-MSH and adenovirus fiber poly A signal
SEQ ID NO:13: DNA coding a modified fiber protein of pWE6.7R-F/asMSHb
SEQ ID NO:14: Synthetic DNA No. 1060 used as antisense primer for PCR amplification of DNA coding a part of adenovirus type 5 fiber and GS linker peptide
SEQ ID NO:15: Synthetic DNA No. 1098 used as antisense primer for PCR amplification of DNA coding a part of adenovirus type 5 fiber and GS linker peptide
SEQ ID NO:16: Synthetic DNA No. 931 used as sense primer for PCR amplification of DNA coding a part of adenovirus type 5 fiber and GS linker peptide
SEQ ID NO:17: DNA coding a modified fiber protein of pWE6.7R-F/gsMSHa
SEQ ID NO:18: DNA coding a modified fiber protein of pWE6.7R-F/gsMSHb
SEQ ID NO:19: Synthetic DNA No. 1089 used as sense primer for PCR amplification of DNA coding K21 linker
SEQ ID NO:20: DNA coding a modified fiber protein of pWE6.7R-F/asK21MSHa
SEQ ID NO:21: DNA coding a modified fiber protein of pWE6.7R-F/gsK21MSHa
SEQ ID NO:22: DNA coding a modified fiber protein of pWE6.7R-F/asK21MSHb
SEQ ID NO:23: DNA coding a modified fiber protein of pWE6.7R-F/gsK21MSHb
SEQ ID NO:24: DNA coding AS linker
SEQ ID NO:25: AS linker peptide
SEQ ID NO:26: DNA coding GS linker
SEQ ID NO:27: GS linker peptide
SEQ ID NO:28: DNA coding asK21 linker
SEQ ID NO:29: asK21 linker peptide
SEQ ID NO:30: DNA coding gsK21 linker
SEQ ID NO:31: gsK21 linker peptide
SEQ ID NO:32: A modified fiber protein encoded in pWE6.7R-F/asMSHa
SEQ ID NO:33: A modified fiber protein encoded in pWE6.7R-F/asMSHb
SEQ ID NO:34: A modified fiber protein encoded in pWE6.7R-F/gsMSHa
SEQ ID NO:35: A modified fiber protein encoded in pWE6.7R-F/gsMSHb
SEQ ID NO:36: A modified fiber protein encoded in pWE6.7R-F/asK21MSHa
SEQ ID NO:37: A modified fiber protein encoded in pWE6.7R-F/gsK21MSHa
SEQ ID NO:38: A modified fiber protein encoded in pWE6.7R-F/asK21MSHb
SEQ ID NO:39: A modified fiber protein encoded in pNE6.7R-F/gsK21MSHb

SEQUENCE LISTING
<110> Juridical Foundation, Japanese Foundation For Cancer Research

<120> vector for gene therapy of malignamt melanoma, with use of virus
having MSH fused protein.

<130> H11-0241J2

<160> 39

<170> PatentIn Ver. 2.0

<210> 1
<211> 166
<212> DNA
<213> Artificial Sequence

<220>
<223> DNA coding a part of adenovirus type 5 fiber, AS linker peptide and
α-MSH.

<220>
<221> CDS
<222> (3)..(113)

<400> 1

gg gaa ttc tcg agt tac act ttt tca tac att gcc caa gaa cca tca     47

Glu Phe Ser Ser Tyr Thr Phe Ser Tyr Ile Ala Gln Glu Pro Ser

1               5                   10                  15

gcc tcc gca tct gct tcc gcc cct gga tcc tac tcc atg gag cac ttc     95

Ala Ser Ala Ser Ala Ser Ala Pro Gly Ser Tyr Ser Met Glu His Phe

                20                  25                  30

cgc tgg ggc aag ccg gtg taaagaatcg tttgtgttat gtttcaacgt     143

Arg Trp Gly Lys Pro Val

            35

gtttattttt caattgaatt ccc     166

<210> 2

<211> 126

<212> DNA

<213> Artificial Sequence

<220>

<223> synthetic DNA No. 924 used as temprate for PCR amplification of DNA

sequence No. 1.

<400> 2

cgttgaaaca taacacaaac gattctttac accggcttgc cccagcggaa gtgctccatg     60

gagtaggatc caggggcgga agcagatgcg gaggctgatg gttcttgggc aatgtatgaa     120

aaagtg     126

<210> 3

<211> 39

<212> DNA

<213> Artificial Sequence

<220>

<223> synthetic DNA No. 933 used as sense primer for PCR amplification of

DNA sequence No. 1.

<400> 3

gggaattctc gagttacact ttttcataca ttgcccaag 39

<210> 4

<211> 49

<212> DNA

<213> Artificial Sequence

<220>

<223> synthetic DNA No. 934 used as antisense primer for PCR amplification

of DNA sequence No. 1.

<400> 4

gggaattcaa ttgaaaaata aacacgttga aacataacac aaacgattc 49

<210> 5

<211> 76

<212> DNA

<213> Artificial Sequence

<220>

<223> synthetic DNA No. 1061 used as sense primer for PCR amplification of

DNA coding α-MSH and adenovirus fiber poly A signal.

<400> 5

cgggatccta ctccatggag cacttccgct ggggcaagcc ggtgtaagtc gacaagaata 60

aagaatcgtt tgtgtt 76

<210> 6

<211> 32

<212> DNA

<213> Artificial Sequence

<220>

<223> synthetic DNA No. 1092 used as antisense primer for PCR amplification

of DNA coding α-MSH and adenovirus fiber poly A signal.

<400> 6

cggaattcat ggcgccatgt ttaatcagag gt 32

<210> 7

<211> 1818

<212> DNA

<213> Artificial Sequence

<220>

<223> DNA coding a modified fiber protein of pWE6.7R-F/asMSHa

<220>

<221> CDS

<222> (1)..(1815)

<400> 7

atg aag cgc gca aga ccg tct gaa gat acc ttc aac ccc gtg tat cca 48

Met Lys Arg Ala Arg Pro Ser Glu Asp Thr Phe Asn Pro Val Tyr Pro

1               5                   10                  15

tat gac acg gaa acc ggt cct cca act gtg cct ttt ctt act cct ccc 96

Tyr Asp Thr Glu Thr Gly Pro Pro Thr Val Pro Phe Leu Thr Pro Pro

            20                  25                  30

ttt gta tcc ccc aat ggg ttt caa gag agt ccc cct ggg gta ctc tct 144

Phe Val Ser Pro Asn Gly Phe Gln Glu Ser Pro Pro Gly Val Leu Ser

        35                  40                  45

ttg cgc cta tcc gaa cct cta gtt acc tcc aat ggc atg ctt gcg ctc 192
Leu Arg Leu Ser Glu Pro Leu Val Thr Ser Asn Gly Met Leu Ala Leu
    50                  55                  60

aaa atg ggc aac ggc ctc tct ctg gac gag gcc ggc aac ctt acc tcc 240
Lys Met Gly Asn Gly Leu Ser Leu Asp Glu Ala Gly Asn Leu Thr Ser
65                  70                  75                  80

caa aat gta acc act gtg agc cca cct ctc aaa aaa acc aag tca aac 288
Gln Asn Val Thr Thr Val Ser Pro Pro Leu Lys Lys Thr Lys Ser Asn
                85                  90                  95

ata aac ctg gaa ata tct gca ccc ctc aca gtt acc tca gaa gcc cta 336
Ile Asn Leu Glu Ile Ser Ala Pro Leu Thr Val Thr Ser Glu Ala Leu
            100                 105                 110

act gtg gct gcc gcc gca cct cta atg gtc gcg ggc aac aca ctc acc 384
Thr Val Ala Ala Ala Ala Pro Leu Met Val Ala Gly Asn Thr Leu Thr
        115                 120                 125

atg caa tca cag gcc ccg cta acc gtg cac gac tcc aaa ctt agc att 432
Met Gln Ser Gln Ala Pro Leu Thr Val His Asp Ser Lys Leu Ser Ile
    130                 135                 140

gcc acc caa gga ccc ctc aca gtg tca gaa gga aag cta gcc ctg caa 480

Ala Thr Gln Gly Pro Leu Thr Val Ser Glu Gly Lys Leu Ala Leu Gln
145                 150                 155                 160

aca tca ggc ccc ctc acc acc acc gat agc agt acc ctt act atc act     528
Thr Ser Gly Pro Leu Thr Thr Thr Asp Ser Ser Thr Leu Thr Ile Thr
                165                 170                 175

gcc tca ccc cct cta act act gcc act ggt agc ttg ggc att gac ttg     576
Ala Ser Pro Pro Leu Thr Thr Ala Thr Gly Ser Leu Gly Ile Asp Leu
            180                 185                 190

aaa gag ccc att tat aca caa aat gga aaa cta gga cta aag tac ggg     624
Lys Glu Pro Ile Tyr Thr Gln Asn Gly Lys Leu Gly Leu Lys Tyr Gly
        195                 200                 205

gct cct ttg cat gta aca gac gac cta aac act ttg acc gta gca act     672
Ala Pro Leu His Val Thr Asp Asp Leu Asn Thr Leu Thr Val Ala Thr
    210                 215                 220

ggt cca ggt gtg act att aat aat act tcc ttg caa act aaa gtt act     720
Gly Pro Gly Val Thr Ile Asn Asn Thr Ser Leu Gln Thr Lys Val Thr
225                 230                 235                 240

gga gcc ttg ggt ttt gat tca caa ggc aat atg caa ctt aat gta gca     768
Gly Ala Leu Gly Phe Asp Ser Gln Gly Asn Met Gln Leu Asn Val Ala
                245                 250                 255

gga gga cta agg att gat tct caa aac aga cgc ctt ata ctt gat gtt 816
Gly Gly Leu Arg Ile Asp Ser Gln Asn Arg Arg Leu Ile Leu Asp Val
            260                 265                 270

agt tat ccg ttt gat gct caa aac caa cta aat cta aga cta gga cag 864
Ser Tyr Pro Phe Asp Ala Gln Asn Gln Leu Asn Leu Arg Leu Gly Gln
        275                 280                 285

ggc cct ctt ttt ata aac tca gcc cac aac ttg gat att aac tac aac 912
Gly Pro Leu Phe Ile Asn Ser Ala His Asn Leu Asp Ile Asn Tyr Asn
    290                 295                 300

aaa ggc ctt tac ttg ttt aca gct tca aac aat tcc aaa aag ctt gag 960
Lys Gly Leu Tyr Leu Phe Thr Ala Ser Asn Asn Ser Lys Lys Leu Glu
305                 310                 315                 320

gtt aac cta agc act gcc aag ggg ttg atg ttt gac gct aca gcc ata 1008
Val Asn Leu Ser Thr Ala Lys Gly Leu Met Phe Asp Ala Thr Ala Ile
                325                 330                 335

gcc att aat gca gga gat ggg ctt gaa ttt ggt tca cct aat gca cca 1056
Ala Ile Asn Ala Gly Asp Gly Leu Glu Phe Gly Ser Pro Asn Ala Pro
            340                 345                 350

aac aca aat ccc ctc aaa aca aaa att ggc cat ggc cta gaa ttt gat 1104

Asn Thr Asn Pro Leu Lys Thr Lys Ile Gly His Gly Leu Glu Phe Asp
        355                 360                 365

tca aac aag gct atg gtt cct aaa cta gga act ggc ctt agt ttt gac 1152
Ser Asn Lys Ala Met Val Pro Lys Leu Gly Thr Gly Leu Ser Phe Asp
    370                 375                 380

agc aca ggt gcc att aca gta gga aac aaa aat aat gat aag cta act 1200
Ser Thr Gly Ala Ile Thr Val Gly Asn Lys Asn Asn Asp Lys Leu Thr
385                 390                 395                 400

ttg tgg acc aca cca gct cca tct cct aac tgt aga cta aat gca gag 1248
Leu Trp Thr Thr Pro Ala Pro Ser Pro Asn Cys Arg Leu Asn Ala Glu
                405                 410                 415

aaa gat gct aaa ctc act ttg gtc tta aca aaa tgt ggc agt caa ata 1296
Lys Asp Ala Lys Leu Thr Leu Val Leu Thr Lys Cys Gly Ser Gln Ile
            420                 425                 430

ctt gct aca gtt tca gtt ttg gct gtt aaa ggc agt ttg gct cca ata 1344
Leu Ala Thr Val Ser Val Leu Ala Val Lys Gly Ser Leu Ala Pro Ile
        435                 440                 445

tct gga aca gtt caa agt gct cat ctt att ata aga ttt gac gaa aat 1392
Ser Gly Thr Val Gln Ser Ala His Leu Ile Ile Arg Phe Asp Glu Asn
    450                 455                 460

gga gtg cta cta aac aat tcc ttc ctg gac cca gaa tat tgg aac ttt 1440
Gly Val Leu Leu Asn Asn Ser Phe Leu Asp Pro Glu Tyr Trp Asn Phe
465                 470                 475                 480

aga aat gga gat ctt act gaa ggc aca gcc tat aca aac gct gtt gga 1488
Arg Asn Gly Asp Leu Thr Glu Gly Thr Ala Tyr Thr Asn Ala Val Gly
                485                 490                 495

ttt atg cct aac cta tca gct tat cca aaa tct cac ggt aaa act gcc 1536
Phe Met Pro Asn Leu Ser Ala Tyr Pro Lys Ser His Gly Lys Thr Ala
            500                 505                 510

aaa agt aac att gtc agt caa gtt tac tta aac gga gac aaa act aaa 1584
Lys Ser Asn Ile Val Ser Gln Val Tyr Leu Asn Gly Asp Lys Thr Lys
        515                 520                 525

cct gta aca cta acc att aca cta aac ggt aca cag gaa aca gga gac 1632
Pro Val Thr Leu Thr Ile Thr Leu Asn Gly Thr Gln Glu Thr Gly Asp
    530                 535                 540

aca act cca agt gca tac tct atg tca ttt tca tgg gac tgg tct ggc 1680
Thr Thr Pro Ser Ala Tyr Ser Met Ser Phe Ser Trp Asp Trp Ser Gly
545                 550                 555                 560

cac aac tac att aat gaa ata ttt gcc acc tcg agt tac act ttt tca 1728

His Asn Tyr Ile Asn Glu Ile Phe Ala Thr Ser Ser Tyr Thr Phe Ser
                565                 570                 575

tac att gcc caa gaa cca tca gcc tcc gca tct gct tcc gcc cct gga 1776
Tyr Ile Ala Gln Glu Pro Ser Ala Ser Ala Ser Ala Ser Ala Pro Gly
            580                 585                 590

tcc tac tcc atg gag cac ttc cgc tgg ggc aag ccg gtg taa 1818
Ser Tyr Ser Met Glu His Phe Arg Trp Gly Lys Pro Val
        595                 600                 605

<210> 8
<211> 40
<212> DNA
<213> Artificial Sequence

<220>
<223> synthetic DNA No. 1037 used as sense primer for PCR amplification of
DNA coding human MSH receptor residue 1-154.

<400> 8
gggaattcac catggctgtg cagggatccc agagaagact 40

<210> 9

<211> 33

<212> DNA

<213> Artificial Sequence

<220>

<223> synthetic DNA No. 1038 used as antisense primer for PCR amplification

of DNA coding human MSH receptor residue 150-317.

<400> 9

gggaattcac caggagcatg tcagcacctc ctt 33

<210> 10

<211> 27

<212> DNA

<213> Artificial Sequence

<220>

<223> synthetic DNA No. 1039 used as sense primer for PCR amplification of

DNA coding human MSH receptor residue 150-317.

<400> 10

ctgcggtacc acagcatcgt gaccctg 27

<210> 11

<211> 27

<212> DNA
<213> Artificial Sequence

<220>
<223> synthetic DNA No. 1040 used as antisense primer for PCR amplification
of DNA coding human MSH receptor residue 1-154.

<400> 11
gctgtggtac cgcagtgcgt agaagat                                          27

<210> 12
<211> 107
<212> DNA
<213> Artificial Sequence

<220>
<223> synthetic DNA No. 1075 used as sense primer for PCR amplification of
DNA coding β-MSH and adenovirus fiber poly A signal.

<400> 12
cgcggatccg ccgagaagaa ggacgagggc ccctacagga tggagcactt ccgctggggc      60
agcccgccca aggactaagt cgacaagaat aaagaatcgt ttgtgtt                   107

<210> 13

<211> 1848

<212> DNA

<213> Artificial Sequence

<220>

<223> DNA coding a modified fiber protein of pWE6.7R-F/asMSHb

<220>

<221> CDS

<222> (1)..(1845)

<400> 13

atg aag cgc gca aga ccg tct gaa gat acc ttc aac ccc gtg tat cca 48

Met Lys Arg Ala Arg Pro Ser Glu Asp Thr Phe Asn Pro Val Tyr Pro

1               5                   10                  15

tat gac acg gaa acc ggt cct cca act gtg cct ttt ctt act cct ccc 96

Tyr Asp Thr Glu Thr Gly Pro Pro Thr Val Pro Phe Leu Thr Pro Pro

            20                  25                  30

ttt gta tcc ccc aat ggg ttt caa gag agt ccc cct ggg gta ctc tct 144

Phe Val Ser Pro Asn Gly Phe Gln Glu Ser Pro Pro Gly Val Leu Ser

        35                  40                  45

ttg cgc cta tcc gaa cct cta gtt acc tcc aat ggc atg ctt gcg ctc 192

Leu Arg Leu Ser Glu Pro Leu Val Thr Ser Asn Gly Met Leu Ala Leu

    50                  55                  60

aaa atg ggc aac ggc ctc tct ctg gac gag gcc ggc aac ctt acc tcc 240
Lys Met Gly Asn Gly Leu Ser Leu Asp Glu Ala Gly Asn Leu Thr Ser
65                  70                  75                  80

caa aat gta acc act gtg agc cca cct ctc aaa aaa acc aag tca aac 288
Gln Asn Val Thr Thr Val Ser Pro Pro Leu Lys Lys Thr Lys Ser Asn
                85                  90                  95

ata aac ctg gaa ata tct gca ccc ctc aca gtt acc tca gaa gcc cta 336
Ile Asn Leu Glu Ile Ser Ala Pro Leu Thr Val Thr Ser Glu Ala Leu
            100                 105                 110

act gtg gct gcc gcc gca cct cta atg gtc gcg ggc aac aca ctc acc 384
Thr Val Ala Ala Ala Ala Pro Leu Met Val Ala Gly Asn Thr Leu Thr
        115                 120                 125

atg caa tca cag gcc ccg cta acc gtg cac gac tcc aaa ctt agc att 432
Met Gln Ser Gln Ala Pro Leu Thr Val His Asp Ser Lys Leu Ser Ile
    130                 135                 140

gcc acc caa gga ccc ctc aca gtg tca gaa gga aag cta gcc ctg caa 480
Ala Thr Gln Gly Pro Leu Thr Val Ser Glu Gly Lys Leu Ala Leu Gln
145                 150                 155                 160

aca tca ggc ccc ctc acc acc acc gat agc agt acc ctt act atc act 528
Thr Ser Gly Pro Leu Thr Thr Thr Asp Ser Ser Thr Leu Thr Ile Thr
                165                 170                 175

gcc tca ccc cct cta act act gcc act ggt agc ttg ggc att gac ttg 576
Ala Ser Pro Pro Leu Thr Thr Ala Thr Gly Ser Leu Gly Ile Asp Leu
            180                 185                 190

aaa gag ccc att tat aca caa aat gga aaa cta gga cta aag tac ggg 624
Lys Glu Pro Ile Tyr Thr Gln Asn Gly Lys Leu Gly Leu Lys Tyr Gly
        195                 200                 205

gct cct ttg cat gta aca gac gac cta aac act ttg acc gta gca act 672
Ala Pro Leu His Val Thr Asp Asp Leu Asn Thr Leu Thr Val Ala Thr
    210                 215                 220

ggt cca ggt gtg act att aat aat act tcc ttg caa act aaa gtt act 720
Gly Pro Gly Val Thr Ile Asn Asn Thr Ser Leu Gln Thr Lys Val Thr
225                 230                 235                 240

gga gcc ttg ggt ttt gat tca caa ggc aat atg caa ctt aat gta gca 768
Gly Ala Leu Gly Phe Asp Ser Gln Gly Asn Met Gln Leu Asn Val Ala
                245                 250                 255

gga gga cta agg att gat tct caa aac aga cgc ctt ata ctt gat gtt 816
Gly Gly Leu Arg Ile Asp Ser Gln Asn Arg Arg Leu Ile Leu Asp Val

            260                 265                 270

agt tat ccg ttt gat gct caa aac caa cta aat cta aga cta gga cag 864
Ser Tyr Pro Phe Asp Ala Gln Asn Gln Leu Asn Leu Arg Leu Gly Gln
        275                 280                 285

ggc cct ctt ttt ata aac tca gcc cac aac ttg gat att aac tac aac 912
Gly Pro Leu Phe Ile Asn Ser Ala His Asn Leu Asp Ile Asn Tyr Asn
    290                 295                 300

aaa ggc ctt tac ttg ttt aca gct tca aac aat tcc aaa aag ctt gag 960
Lys Gly Leu Tyr Leu Phe Thr Ala Ser Asn Asn Ser Lys Lys Leu Glu
305                 310                 315                 320

gtt aac cta agc act gcc aag ggg ttg atg ttt gac gct aca gcc ata 1008
Val Asn Leu Ser Thr Ala Lys Gly Leu Met Phe Asp Ala Thr Ala Ile
                325                 330                 335

gcc att aat gca gga gat ggg ctt gaa ttt ggt tca cct aat gca cca 1056
Ala Ile Asn Ala Gly Asp Gly Leu Glu Phe Gly Ser Pro Asn Ala Pro
            340                 345                 350

aac aca aat ccc ctc aaa aca aaa att ggc cat ggc cta gaa ttt gat 1104
Asn Thr Asn Pro Leu Lys Thr Lys Ile Gly His Gly Leu Glu Phe Asp
        355                 360                 365

tca aac aag gct atg gtt cct aaa cta gga act ggc ctt agt ttt gac 1152
Ser Asn Lys Ala Met Val Pro Lys Leu Gly Thr Gly Leu Ser Phe Asp
    370                 375                 380

agc aca ggt gcc att aca gta gga aac aaa aat aat gat aag cta act 1200
Ser Thr Gly Ala Ile Thr Val Gly Asn Lys Asn Asn Asp Lys Leu Thr
385                 390                 395                 400

ttg tgg acc aca cca gct cca tct cct aac tgt aga cta aat gca gag 1248
Leu Trp Thr Thr Pro Ala Pro Ser Pro Asn Cys Arg Leu Asn Ala Glu
                405                 410                 415

aaa gat gct aaa ctc act ttg gtc tta aca aaa tgt ggc agt caa ata 1296
Lys Asp Ala Lys Leu Thr Leu Val Leu Thr Lys Cys Gly Ser Gln Ile
            420                 425                 430

ctt gct aca gtt tca gtt ttg gct gtt aaa ggc agt ttg gct cca ata 1344
Leu Ala Thr Val Ser Val Leu Ala Val Lys Gly Ser Leu Ala Pro Ile
        435                 440                 445

tct gga aca gtt caa agt gct cat ctt att ata aga ttt gac gaa aat 1392
Ser Gly Thr Val Gln Ser Ala His Leu Ile Ile Arg Phe Asp Glu Asn
    450                 455                 460

gga gtg cta cta aac aat tcc ttc ctg gac cca gaa tat tgg aac ttt 1440
Gly Val Leu Leu Asn Asn Ser Phe Leu Asp Pro Glu Tyr Trp Asn Phe

465                 470                 475                 480

aga aat gga gat ctt act gaa ggc aca gcc tat aca aac gct gtt gga 1488
Arg Asn Gly Asp Leu Thr Glu Gly Thr Ala Tyr Thr Asn Ala Val Gly
                485                 490                 495

ttt atg cct aac cta tca gct tat cca aaa tct cac ggt aaa act gcc 1536
Phe Met Pro Asn Leu Ser Ala Tyr Pro Lys Ser His Gly Lys Thr Ala
            500                 505                 510

aaa agt aac att gtc agt caa gtt tac tta aac gga gac aaa act aaa 1584
Lys Ser Asn Ile Val Ser Gln Val Tyr Leu Asn Gly Asp Lys Thr Lys
        515                 520                 525

cct gta aca cta acc att aca cta aac ggt aca cag gaa aca gga gac 1632
Pro Val Thr Leu Thr Ile Thr Leu Asn Gly Thr Gln Glu Thr Gly Asp
    530                 535                 540

aca act cca agt gca tac tct atg tca ttt tca tgg gac tgg tct ggc 1680
Thr Thr Pro Ser Ala Tyr Ser Met Ser Phe Ser Trp Asp Trp Ser Gly
545                 550                 555                 560

cac aac tac att aat gaa ata ttt gcc acc tcg agt tac act ttt tca 1728
His Asn Tyr Ile Asn Glu Ile Phe Ala Thr Ser Ser Tyr Thr Phe Ser
                565                 570                 575

tac att gcc caa gaa cca tca gcc tcc gca tct gct tcc gcc cct gga 1776
Tyr Ile Ala Gln Glu Pro Ser Ala Ser Ala Ser Ala Ser Ala Pro Gly
            580                 585                 590

tcc gcc gag aag aag gac gag ggc ccc tac agg atg gag cac ttc cgc 1824
Ser Ala Glu Lys Lys Asp Glu Gly Pro Tyr Arg Met Glu His Phe Arg
        595                 600                 605

tgg ggc agc ccg ccc aag gac taa 1848
Trp Gly Ser Pro Pro Lys Asp
    610                 615

<210> 14
<211> 61
<212> DNA
<213> Artificial Sequence

<220>
<223> synthetic DNA No. 1060 used as antisense primer for PCR amplification
of DNA coding a part of adenovirus type 5 fiber and GS linker peptide.

<400> 14
cgggatccag atccagaacc actaccactt ccagaacctt cttgggcaat gtatgaaaaa 60
g 61

<210> 15
<211> 41
<212> DNA
<213> Artificial Sequence

<220>
<223> synthetic DNA No. 1098 used as antisense primer for PCR amplification
of DNA coding a part of adenovirus type 5 fiber and GS linker peptide.

<400> 15
cgtgtggatc cgctgccaga accactacca cttccagaac c                41

<210> 16
<211> 20
<212> DNA
<213> Artificial Sequence

<220>
<223> synthetic DNA No. 931 used as sense primer for PCR amplification of
DNA coding a part of adenovirus type 5 fiber and GS linker peptide.

<400> 16
ggcctttact tgtttacagc                20

<210> 17

<211> 1818

<212> DNA

<213> Artificial Sequence

<220>

<223> DNA coding a modified fiber protein of pWE6.7R-F/gsMSHa

<220>

<221> CDS

<222> (1)..(1815)

<400> 17

atg aag cgc gca aga ccg tct gaa gat acc ttc aac ccc gtg tat cca 48

Met Lys Arg Ala Arg Pro Ser Glu Asp Thr Phe Asn Pro Val Tyr Pro

1               5                   10                  15

tat gac acg gaa acc ggt cct cca act gtg cct ttt ctt act cct ccc 96

Tyr Asp Thr Glu Thr Gly Pro Pro Thr Val Pro Phe Leu Thr Pro Pro

            20                  25                  30

ttt gta tcc ccc aat ggg ttt caa gag agt ccc cct ggg gta ctc tct 144

Phe Val Ser Pro Asn Gly Phe Gln Glu Ser Pro Pro Gly Val Leu Ser

        35                  40                  45

ttg cgc cta tcc gaa cct cta gtt acc tcc aat ggc atg ctt gcg ctc 192

Leu Arg Leu Ser Glu Pro Leu Val Thr Ser Asn Gly Met Leu Ala Leu
    50                  55                  60

aaa atg ggc aac ggc ctc tct ctg gac gag gcc ggc aac ctt acc tcc 240
Lys Met Gly Asn Gly Leu Ser Leu Asp Glu Ala Gly Asn Leu Thr Ser
65                  70                  75                  80

caa aat gta acc act gtg agc cca cct ctc aaa aaa acc aag tca aac 288
Gln Asn Val Thr Thr Val Ser Pro Pro Leu Lys Lys Thr Lys Ser Asn
                85                  90                  95

ata aac ctg gaa ata tct gca ccc ctc aca gtt acc tca gaa gcc cta 336
Ile Asn Leu Glu Ile Ser Ala Pro Leu Thr Val Thr Ser Glu Ala Leu
            100                 105                 110

act gtg gct gcc gcc gca cct cta atg gtc gcg ggc aac aca ctc acc 384
Thr Val Ala Ala Ala Ala Pro Leu Met Val Ala Gly Asn Thr Leu Thr
        115                 120                 125

atg caa tca cag gcc ccg cta acc gtg cac gac tcc aaa ctt agc att 432
Met Gln Ser Gln Ala Pro Leu Thr Val His Asp Ser Lys Leu Ser Ile
    130                 135                 140

gcc acc caa gga ccc ctc aca gtg tca gaa gga aag cta gcc ctg caa 480
Ala Thr Gln Gly Pro Leu Thr Val Ser Glu Gly Lys Leu Ala Leu Gln
145                 150                 155                 160

aca tca ggc ccc ctc acc acc acc gat agc agt acc ctt act atc act 528
Thr Ser Gly Pro Leu Thr Thr Thr Asp Ser Ser Thr Leu Thr Ile Thr
                165                 170                 175

gcc tca ccc cct cta act act gcc act ggt agc ttg ggc att gac ttg 576
Ala Ser Pro Pro Leu Thr Thr Ala Thr Gly Ser Leu Gly Ile Asp Leu
            180                 185                 190

aaa gag ccc att tat aca caa aat gga aaa cta gga cta aag tac ggg 624
Lys Glu Pro Ile Tyr Thr Gln Asn Gly Lys Leu Gly Leu Lys Tyr Gly
        195                 200                 205

gct cct ttg cat gta aca gac gac cta aac act ttg acc gta gca act 672
Ala Pro Leu His Val Thr Asp Asp Leu Asn Thr Leu Thr Val Ala Thr
    210                 215                 220

ggt cca ggt gtg act att aat aat act tcc ttg caa act aaa gtt act 720
Gly Pro Gly Val Thr Ile Asn Asn Thr Ser Leu Gln Thr Lys Val Thr
225                 230                 235                 240

gga gcc ttg ggt ttt gat tca caa ggc aat atg caa ctt aat gta gca 768
Gly Ala Leu Gly Phe Asp Ser Gln Gly Asn Met Gln Leu Asn Val Ala
                245                 250                 255

gga gga cta agg att gat tct caa aac aga cgc ctt ata ctt gat gtt 816

Gly Gly Leu Arg Ile Asp Ser Gln Asn Arg Arg Leu Ile Leu Asp Val
            260                 265                 270

agt tat ccg ttt gat gct caa aac caa cta aat cta aga cta gga cag 864
Ser Tyr Pro Phe Asp Ala Gln Asn Gln Leu Asn Leu Arg Leu Gly Gln
        275                 280                 285

ggc cct ctt ttt ata aac tca gcc cac aac ttg gat att aac tac aac 912
Gly Pro Leu Phe Ile Asn Ser Ala His Asn Leu Asp Ile Asn Tyr Asn
    290                 295                 300

aaa ggc ctt tac ttg ttt aca gct tca aac aat tcc aaa aag ctt gag 960
Lys Gly Leu Tyr Leu Phe Thr Ala Ser Asn Asn Ser Lys Lys Leu Glu
305                 310                 315                 320

gtt aac cta agc act gcc aag ggg ttg atg ttt gac gct aca gcc ata 1008
Val Asn Leu Ser Thr Ala Lys Gly Leu Met Phe Asp Ala Thr Ala Ile
                325                 330                 335

gcc att aat gca gga gat ggg ctt gaa ttt ggt tca cct aat gca cca 1056
Ala Ile Asn Ala Gly Asp Gly Leu Glu Phe Gly Ser Pro Asn Ala Pro
            340                 345                 350

aac aca aat ccc ctc aaa aca aaa att ggc cat ggc cta gaa ttt gat 1104
Asn Thr Asn Pro Leu Lys Thr Lys Ile Gly His Gly Leu Glu Phe Asp
        355                 360                 365

tca aac aag gct atg gtt cct aaa cta gga act ggc ctt agt ttt gac 1152
Ser Asn Lys Ala Met Val Pro Lys Leu Gly Thr Gly Leu Ser Phe Asp
    370                 375                 380

agc aca ggt gcc att aca gta gga aac aaa aat aat gat aag cta act 1200
Ser Thr Gly Ala Ile Thr Val Gly Asn Lys Asn Asn Asp Lys Leu Thr
385                 390                 395                 400

ttg tgg acc aca cca gct cca tct cct aac tgt aga cta aat gca gag 1248
Leu Trp Thr Thr Pro Ala Pro Ser Pro Asn Cys Arg Leu Asn Ala Glu
                405                 410                 415

aaa gat gct aaa ctc act ttg gtc tta aca aaa tgt ggc agt caa ata 1296
Lys Asp Ala Lys Leu Thr Leu Val Leu Thr Lys Cys Gly Ser Gln Ile
            420                 425                 430

ctt gct aca gtt tca gtt ttg gct gtt aaa ggc agt ttg gct cca ata 1344
Leu Ala Thr Val Ser Val Leu Ala Val Lys Gly Ser Leu Ala Pro Ile
        435                 440                 445

tct gga aca gtt caa agt gct cat ctt att ata aga ttt gac gaa aat 1392
Ser Gly Thr Val Gln Ser Ala His Leu Ile Ile Arg Phe Asp Glu Asn
    450                 455                 460

gga gtg cta cta aac aat tcc ttc ctg gac cca gaa tat tgg aac ttt 1440

Gly Val Leu Leu Asn Asn Ser Phe Leu Asp Pro Glu Tyr Trp Asn Phe
465                 470                 475                 480

aga aat gga gat ctt act gaa ggc aca gcc tat aca aac gct gtt gga 1488
Arg Asn Gly Asp Leu Thr Glu Gly Thr Ala Tyr Thr Asn Ala Val Gly
                485                 490                 495

ttt atg cct aac cta tca gct tat cca aaa tct cac ggt aaa act gcc 1536
Phe Met Pro Asn Leu Ser Ala Tyr Pro Lys Ser His Gly Lys Thr Ala
            500                 505                 510

aaa agt aac att gtc agt caa gtt tac tta aac gga gac aaa act aaa 1584
Lys Ser Asn Ile Val Ser Gln Val Tyr Leu Asn Gly Asp Lys Thr Lys
        515                 520                 525

cct gta aca cta acc att aca cta aac ggt aca cag gaa aca gga gac 1632
Pro Val Thr Leu Thr Ile Thr Leu Asn Gly Thr Gln Glu Thr Gly Asp
    530                 535                 540

aca act cca agt gca tac tct atg tca ttt tca tgg gac tgg tct ggc 1680
Thr Thr Pro Ser Ala Tyr Ser Met Ser Phe Ser Trp Asp Trp Ser Gly
545                 550                 555                 560

cac aac tac att aat gaa ata ttt gcc acc tcg agt tac act ttt tca 1728
His Asn Tyr Ile Asn Glu Ile Phe Ala Thr Ser Ser Tyr Thr Phe Ser
                565                 570                 575

tac att gcc caa gaa ggt tct gga agt ggt agt ggt tct ggc agc gga 1776
Tyr Ile Ala Gln Glu Gly Ser Gly Ser Gly Ser Gly Ser Gly Ser Gly
            580                 585                 590

tcc tac tcc atg gag cac ttc cgc tgg ggc aag ccg gtg taa 1818
Ser Tyr Ser Met Glu His Phe Arg Trp Gly Lys Pro Val
        595                 600                 605

<210> 18
<211> 1848
<212> DNA
<213> Artificial Sequence

<220>
<223> DNA coding a modified fiber protein of pWE6.7R-F/gsMSHb

<220>
<221> CDS
<222> (1)..(1845)

<400> 18
atg aag cgc gca aga ccg tct gaa gat acc ttc aac ccc gtg tat cca 48
Met Lys Arg Ala Arg Pro Ser Glu Asp Thr Phe Asn Pro Val Tyr Pro
1               5                   10                  15

tat gac acg gaa acc ggt cct cca act gtg cct ttt ctt act cct ccc 96
Tyr Asp Thr Glu Thr Gly Pro Pro Thr Val Pro Phe Leu Thr Pro Pro
            20                  25                  30
ttt gta tcc ccc aat ggg ttt caa gag agt ccc cct ggg gta ctc tct 144
Phe Val Ser Pro Asn Gly Phe Gln Glu Ser Pro Pro Gly Val Leu Ser
        35                  40                  45
ttg cgc cta tcc gaa cct cta gtt acc tcc aat ggc atg ctt gcg ctc 192
Leu Arg Leu Ser Glu Pro Leu Val Thr Ser Asn Gly Met Leu Ala Leu
    50                  55                  60
aaa atg ggc aac ggc ctc tct ctg gac gag gcc ggc aac ctt acc tcc 240
Lys Met Gly Asn Gly Leu Ser Leu Asp Glu Ala Gly Asn Leu Thr Ser
65                  70                  75                  80
caa aat gta acc act gtg agc cca cct ctc aaa aaa acc aag tca aac 288
Gln Asn Val Thr Thr Val Ser Pro Pro Leu Lys Lys Thr Lys Ser Asn
                85                  90                  95
ata aac ctg gaa ata tct gca ccc ctc aca gtt acc tca gaa gcc cta 336
Ile Asn Leu Glu Ile Ser Ala Pro Leu Thr Val Thr Ser Glu Ala Leu
            100                 105                 110
act gtg gct gcc gcc gca cct cta atg gtc gcg ggc aac aca ctc acc 384

Thr Val Ala Ala Ala Ala Pro Leu Met Val Ala Gly Asn Thr Leu Thr
        115                 120                 125

atg caa tca cag gcc ccg cta acc gtg cac gac tcc aaa ctt agc att 432
Met Gln Ser Gln Ala Pro Leu Thr Val His Asp Ser Lys Leu Ser Ile
    130                 135                 140

gcc acc caa gga ccc ctc aca gtg tca gaa gga aag cta gcc ctg caa 480
Ala Thr Gln Gly Pro Leu Thr Val Ser Glu Gly Lys Leu Ala Leu Gln
145                 150                 155                 160

aca tca ggc ccc ctc acc acc acc gat agc agt acc ctt act atc act 528
Thr Ser Gly Pro Leu Thr Thr Thr Asp Ser Ser Thr Leu Thr Ile Thr
                165                 170                 175

gcc tca ccc cct cta act act gcc act ggt agc ttg ggc att gac ttg 576
Ala Ser Pro Pro Leu Thr Thr Ala Thr Gly Ser Leu Gly Ile Asp Leu
            180                 185                 190

aaa gag ccc att tat aca caa aat gga aaa cta gga cta aag tac ggg 624
Lys Glu Pro Ile Tyr Thr Gln Asn Gly Lys Leu Gly Leu Lys Tyr Gly
        195                 200                 205

gct cct ttg cat gta aca gac gac cta aac act ttg acc gta gca act 672
Ala Pro Leu His Val Thr Asp Asp Leu Asn Thr Leu Thr Val Ala Thr
    210                 215                 220

ggt cca ggt gtg act att aat aat act tcc ttg caa act aaa gtt act 720
Gly Pro Gly Val Thr Ile Asn Asn Thr Ser Leu Gln Thr Lys Val Thr
225                 230                 235                 240

gga gcc ttg ggt ttt gat tca caa ggc aat atg caa ctt aat gta gca 768
Gly Ala Leu Gly Phe Asp Ser Gln Gly Asn Met Gln Leu Asn Val Ala
                245                 250                 255

gga gga cta agg att gat tct caa aac aga cgc ctt ata ctt gat gtt 816
Gly Gly Leu Arg Ile Asp Ser Gln Asn Arg Arg Leu Ile Leu Asp Val
            260                 265                 270

agt tat ccg ttt gat gct caa aac caa cta aat cta aga cta gga cag 864
Ser Tyr Pro Phe Asp Ala Gln Asn Gln Leu Asn Leu Arg Leu Gly Gln
        275                 280                 285

ggc cct ctt ttt ata aac tca gcc cac aac ttg gat att aac tac aac 912
Gly Pro Leu Phe Ile Asn Ser Ala His Asn Leu Asp Ile Asn Tyr Asn
    290                 295                 300

aaa ggc ctt tac ttg ttt aca gct tca aac aat tcc aaa aag ctt gag 960
Lys Gly Leu Tyr Leu Phe Thr Ala Ser Asn Asn Ser Lys Lys Leu Glu
305                 310                 315                 320

gtt aac cta agc act gcc aag ggg ttg atg ttt gac gct aca gcc ata 1008

Val Asn Leu Ser Thr Ala Lys Gly Leu Met Phe Asp Ala Thr Ala Ile
                325                 330                 335

gcc att aat gca gga gat ggg ctt gaa ttt ggt tca cct aat gca cca 1056
Ala Ile Asn Ala Gly Asp Gly Leu Glu Phe Gly Ser Pro Asn Ala Pro
            340                 345                 350

aac aca aat ccc ctc aaa aca aaa att ggc cat ggc cta gaa ttt gat 1104
Asn Thr Asn Pro Leu Lys Thr Lys Ile Gly His Gly Leu Glu Phe Asp
        355                 360                 365

tca aac aag gct atg gtt cct aaa cta gga act ggc ctt agt ttt gac 1152
Ser Asn Lys Ala Met Val Pro Lys Leu Gly Thr Gly Leu Ser Phe Asp
    370                 375                 380

agc aca ggt gcc att aca gta gga aac aaa aat aat gat aag cta act 1200
Ser Thr Gly Ala Ile Thr Val Gly Asn Lys Asn Asn Asp Lys Leu Thr
385                 390                 395                 400

ttg tgg acc aca cca gct cca tct cct aac tgt aga cta aat gca gag 1248
Leu Trp Thr Thr Pro Ala Pro Ser Pro Asn Cys Arg Leu Asn Ala Glu
                405                 410                 415

aaa gat gct aaa ctc act ttg gtc tta aca aaa tgt ggc agt caa ata 1296
Lys Asp Ala Lys Leu Thr Leu Val Leu Thr Lys Cys Gly Ser Gln Ile
            420                 425                 430

ctt gct aca gtt tca gtt ttg gct gtt aaa ggc agt ttg gct cca ata 1344
Leu Ala Thr Val Ser Val Leu Ala Val Lys Gly Ser Leu Ala Pro Ile
        435                 440                 445

tct gga aca gtt caa agt gct cat ctt att ata aga ttt gac gaa aat 1392
Ser Gly Thr Val Gln Ser Ala His Leu Ile Ile Arg Phe Asp Glu Asn
    450                 455                 460

gga gtg cta cta aac aat tcc ttc ctg gac cca gaa tat tgg aac ttt 1440
Gly Val Leu Leu Asn Asn Ser Phe Leu Asp Pro Glu Tyr Trp Asn Phe
465                 470                 475                 480

aga aat gga gat ctt act gaa ggc aca gcc tat aca aac gct gtt gga 1488
Arg Asn Gly Asp Leu Thr Glu Gly Thr Ala Tyr Thr Asn Ala Val Gly
                485                 490                 495

ttt atg cct aac cta tca gct tat cca aaa tct cac ggt aaa act gcc 1536
Phe Met Pro Asn Leu Ser Ala Tyr Pro Lys Ser His Gly Lys Thr Ala
            500                 505                 510

aaa agt aac att gtc agt caa gtt tac tta aac gga gac aaa act aaa 1584
Lys Ser Asn Ile Val Ser Gln Val Tyr Leu Asn Gly Asp Lys Thr Lys
        515                 520                 525

cct gta aca cta acc att aca cta aac ggt aca cag gaa aca gga gac 1632

Pro Val Thr Leu Thr Ile Thr Leu Asn Gly Thr Gln Glu Thr Gly Asp
    530                 535                 540

aca act cca agt gca tac tct atg tca ttt tca tgg gac tgg tct ggc 1680
Thr Thr Pro Ser Ala Tyr Ser Met Ser Phe Ser Trp Asp Trp Ser Gly
545                 550                 555                 560

cac aac tac att aat gaa ata ttt gcc acc tcg agt tac act ttt tca 1728
His Asn Tyr Ile Asn Glu Ile Phe Ala Thr Ser Ser Tyr Thr Phe Ser
                565                 570                 575

tac att gcc caa gaa ggt tct gga agt ggt agt ggt tct ggc agc gga 1776
Tyr Ile Ala Gln Glu Gly Ser Gly Ser Gly Ser Gly Ser Gly Ser Gly
            580                 585                 590

tcc gcc gag aag aag gac gag ggc ccc tac agg atg gag cac ttc cgc 1824
Ser Ala Glu Lys Lys Asp Glu Gly Pro Tyr Arg Met Glu His Phe Arg
        595                 600                 605

tgg ggc agc ccg ccc aag gac taa 1848
Trp Gly Ser Pro Pro Lys Asp
    610                 615

<210> 19
<211> 128

<212> DNA
<213> Artificial Sequence

<220>
<223> synthetic DNA No. 1089 used as sense primer for PCR amplification of
DNA coding K21 linker peptide.

<400> 19
ccggaattca gatctggatc taagaagaag aagaagaaaa agaagaaaaa gaagaagaag 60
aaaaaaaaga agaagaaaaa gaaaggatcc taagatatcg tcgacaagaa taaagaatcg 120
tttgtgtt 128

<210> 20
<211> 1893
<212> DNA
<213> Artificial Sequence

<220>
<223> DNA coding a modified fiber protein of pWE6.7R-F/asK21MSHa

<220>
<221> CDS
<222> (1)..(1890)

<400> 20

atg aag cgc gca aga ccg tct gaa gat acc ttc aac ccc gtg tat cca 48
Met Lys Arg Ala Arg Pro Ser Glu Asp Thr Phe Asn Pro Val Tyr Pro
1               5                   10                  15

tat gac acg gaa acc ggt cct cca act gtg cct ttt ctt act cct ccc 96
Tyr Asp Thr Glu Thr Gly Pro Pro Thr Val Pro Phe Leu Thr Pro Pro
            20                  25                  30

ttt gta tcc ccc aat ggg ttt caa gag agt ccc cct ggg gta ctc tct 144
Phe Val Ser Pro Asn Gly Phe Gln Glu Ser Pro Pro Gly Val Leu Ser
        35                  40                  45

ttg cgc cta tcc gaa cct cta gtt acc tcc aat ggc atg ctt gcg ctc 192
Leu Arg Leu Ser Glu Pro Leu Val Thr Ser Asn Gly Met Leu Ala Leu
    50                  55                  60

aaa atg ggc aac ggc ctc tct ctg gac gag gcc ggc aac ctt acc tcc 240
Lys Met Gly Asn Gly Leu Ser Leu Asp Glu Ala Gly Asn Leu Thr Ser
65                  70                  75                  80

caa aat gta acc act gtg agc cca cct ctc aaa aaa acc aag tca aac 288
Gln Asn Val Thr Thr Val Ser Pro Pro Leu Lys Lys Thr Lys Ser Asn
                85                  90                  95

ata aac ctg gaa ata tct gca ccc ctc aca gtt acc tca gaa gcc cta 336
Ile Asn Leu Glu Ile Ser Ala Pro Leu Thr Val Thr Ser Glu Ala Leu

            100                 105                 110

act gtg gct gcc gcc gca cct cta atg gtc gcg ggc aac aca ctc acc 384
Thr Val Ala Ala Ala Ala Pro Leu Met Val Ala Gly Asn Thr Leu Thr
        115                 120                 125

atg caa tca cag gcc ccg cta acc gtg cac gac tcc aaa ctt agc att 432
Met Gln Ser Gln Ala Pro Leu Thr Val His Asp Ser Lys Leu Ser Ile
    130                 135                 140

gcc acc caa gga ccc ctc aca gtg tca gaa gga aag cta gcc ctg caa 480
Ala Thr Gln Gly Pro Leu Thr Val Ser Glu Gly Lys Leu Ala Leu Gln
145                 150                 155                 160

aca tca ggc ccc ctc acc acc acc gat agc agt acc ctt act atc act 528
Thr Ser Gly Pro Leu Thr Thr Thr Asp Ser Ser Thr Leu Thr Ile Thr
                165                 170                 175

gcc tca ccc cct cta act act gcc act ggt agc ttg ggc att gac ttg 576
Ala Ser Pro Pro Leu Thr Thr Ala Thr Gly Ser Leu Gly Ile Asp Leu
            180                 185                 190

aaa gag ccc att tat aca caa aat gga aaa cta gga cta aag tac ggg 624
Lys Glu Pro Ile Tyr Thr Gln Asn Gly Lys Leu Gly Leu Lys Tyr Gly
        195                 200                 205

gct cct ttg cat gta aca gac gac cta aac act ttg acc gta gca act 672
Ala Pro Leu His Val Thr Asp Asp Leu Asn Thr Leu Thr Val Ala Thr
    210                 215                 220

ggt cca ggt gtg act att aat aat act tcc ttg caa act aaa gtt act 720
Gly Pro Gly Val Thr Ile Asn Asn Thr Ser Leu Gln Thr Lys Val Thr
225                 230                 235                 240

gga gcc ttg ggt ttt gat tca caa ggc aat atg caa ctt aat gta gca 768
Gly Ala Leu Gly Phe Asp Ser Gln Gly Asn Met Gln Leu Asn Val Ala
                245                 250                 255

gga gga cta agg att gat tct caa aac aga cgc ctt ata ctt gat gtt 816
Gly Gly Leu Arg Ile Asp Ser Gln Asn Arg Arg Leu Ile Leu Asp Val
            260                 265                 270

agt tat ccg ttt gat gct caa aac caa cta aat cta aga cta gga cag 864
Ser Tyr Pro Phe Asp Ala Gln Asn Gln Leu Asn Leu Arg Leu Gly Gln
        275                 280                 285

ggc cct ctt ttt ata aac tca gcc cac aac ttg gat att aac tac aac 912
Gly Pro Leu Phe Ile Asn Ser Ala His Asn Leu Asp Ile Asn Tyr Asn
    290                 295                 300

aaa ggc ctt tac ttg ttt aca gct tca aac aat tcc aaa aag ctt gag 960
Lys Gly Leu Tyr Leu Phe Thr Ala Ser Asn Asn Ser Lys Lys Leu Glu

305                 310                 315                 320

gtt aac cta agc act gcc aag ggg ttg atg ttt gac gct aca gcc ata 1008
Val Asn Leu Ser Thr Ala Lys Gly Leu Met Phe Asp Ala Thr Ala Ile
                325                 330                 335

gcc att aat gca gga gat ggg ctt gaa ttt ggt tca cct aat gca cca 1056
Ala Ile Asn Ala Gly Asp Gly Leu Glu Phe Gly Ser Pro Asn Ala Pro
            340                 345                 350

aac aca aat ccc ctc aaa aca aaa att ggc cat ggc cta gaa ttt gat 1104
Asn Thr Asn Pro Leu Lys Thr Lys Ile Gly His Gly Leu Glu Phe Asp
        355                 360                 365

tca aac aag gct atg gtt cct aaa cta gga act ggc ctt agt ttt gac 1152
Ser Asn Lys Ala Met Val Pro Lys Leu Gly Thr Gly Leu Ser Phe Asp
    370                 375                 380

agc aca ggt gcc att aca gta gga aac aaa aat aat gat aag cta act 1200
Ser Thr Gly Ala Ile Thr Val Gly Asn Lys Asn Asn Asp Lys Leu Thr
385                 390                 395                 400

ttg tgg acc aca cca gct cca tct cct aac tgt aga cta aat gca gag 1248
Leu Trp Thr Thr Pro Ala Pro Ser Pro Asn Cys Arg Leu Asn Ala Glu
                405                 410                 415

aaa gat gct aaa ctc act ttg gtc tta aca aaa tgt ggc agt caa ata 1296
Lys Asp Ala Lys Leu Thr Leu Val Leu Thr Lys Cys Gly Ser Gln Ile
            420                 425                 430

ctt gct aca gtt tca gtt ttg gct gtt aaa ggc agt ttg gct cca ata 1344
Leu Ala Thr Val Ser Val Leu Ala Val Lys Gly Ser Leu Ala Pro Ile
        435                 440                 445

tct gga aca gtt caa agt gct cat ctt att ata aga ttt gac gaa aat 1392
Ser Gly Thr Val Gln Ser Ala His Leu Ile Ile Arg Phe Asp Glu Asn
    450                 455                 460

gga gtg cta cta aac aat tcc ttc ctg gac cca gaa tat tgg aac ttt 1440
Gly Val Leu Leu Asn Asn Ser Phe Leu Asp Pro Glu Tyr Trp Asn Phe
465                 470                 475                 480

aga aat gga gat ctt act gaa ggc aca gcc tat aca aac gct gtt gga 1488
Arg Asn Gly Asp Leu Thr Glu Gly Thr Ala Tyr Thr Asn Ala Val Gly
                485                 490                 495

ttt atg cct aac cta tca gct tat cca aaa tct cac ggt aaa act gcc 1536
Phe Met Pro Asn Leu Ser Ala Tyr Pro Lys Ser His Gly Lys Thr Ala
            500                 505                 510

aaa agt aac att gtc agt caa gtt tac tta aac gga gac aaa act aaa 1584
Lys Ser Asn Ile Val Ser Gln Val Tyr Leu Asn Gly Asp Lys Thr Lys

        515                 520                 525

cct gta aca cta acc att aca cta aac ggt aca cag gaa aca gga gac 1632
Pro Val Thr Leu Thr Ile Thr Leu Asn Gly Thr Gln Glu Thr Gly Asp
    530                 535                 540

aca act cca agt gca tac tct atg tca ttt tca tgg gac tgg tct ggc 1680
Thr Thr Pro Ser Ala Tyr Ser Met Ser Phe Ser Trp Asp Trp Ser Gly
545                 550                 555                 560

cac aac tac att aat gaa ata ttt gcc acc tcg agt tac act ttt tca 1728
His Asn Tyr Ile Asn Glu Ile Phe Ala Thr Ser Ser Tyr Thr Phe Ser
                565                 570                 575

tac att gcc caa gaa cca tca gcc tcc gca tct gct tcc gcc cct gga 1776
Tyr Ile Ala Gln Glu Pro Ser Ala Ser Ala Ser Ala Ser Ala Pro Gly
            580                 585                 590

tct gga tct aag aag aag aag aag aaa aag aag aaa aag aag aag aag 1824
Ser Gly Ser Lys Lys Lys Lys Lys Lys Lys Lys Lys Lys Lys Lys Lys
        595                 600                 605

aaa aaa aag aag aag aaa aag aaa gga tcc tac tcc atg gag cac ttc 1872
Lys Lys Lys Lys Lys Lys Lys Lys Gly Ser Tyr Ser Met Glu His Phe
    610                 615                 620

cgc tgg ggc aag ccg gtg taa 1893
Arg Trp Gly Lys Pro Val
625                 630

<210> 21
<211> 1893
<212> DNA
<213> Artificial Sequence

<220>
<223> DNA coding a modified fiber protein of pWE6.7R-F/gsK21MSHa

<220>
<221> CDS
<222> (1)..(1890)

<400> 21
atg aag cgc gca aga ccg tct gaa gat acc ttc aac ccc gtg tat cca 48
Met Lys Arg Ala Arg Pro Ser Glu Asp Thr Phe Asn Pro Val Tyr Pro
1               5                   10                  15

tat gac acg gaa acc ggt cct cca act gtg cct ttt ctt act cct ccc 96
Tyr Asp Thr Glu Thr Gly Pro Pro Thr Val Pro Phe Leu Thr Pro Pro
            20                  25                  30

ttt gta tcc ccc aat ggg ttt caa gag agt ccc cct ggg gta ctc tct 144
Phe Val Ser Pro Asn Gly Phe Gln Glu Ser Pro Pro Gly Val Leu Ser
        35                  40                  45

ttg cgc cta tcc gaa cct cta gtt acc tcc aat ggc atg ctt gcg ctc 192
Leu Arg Leu Ser Glu Pro Leu Val Thr Ser Asn Gly Met Leu Ala Leu
    50                  55                  60

aaa atg ggc aac ggc ctc tct ctg gac gag gcc ggc aac ctt acc tcc 240
Lys Met Gly Asn Gly Leu Ser Leu Asp Glu Ala Gly Asn Leu Thr Ser
65                  70                  75                  80

caa aat gta acc act gtg agc cca cct ctc aaa aaa acc aag tca aac 288
Gln Asn Val Thr Thr Val Ser Pro Pro Leu Lys Lys Thr Lys Ser Asn
                85                  90                  95

ata aac ctg gaa ata tct gca ccc ctc aca gtt acc tca gaa gcc cta 336
Ile Asn Leu Glu Ile Ser Ala Pro Leu Thr Val Thr Ser Glu Ala Leu
            100                 105                 110

act gtg gct gcc gcc gca cct cta atg gtc gcg ggc aac aca ctc acc 384
Thr Val Ala Ala Ala Ala Pro Leu Met Val Ala Gly Asn Thr Leu Thr
        115                 120                 125

atg caa tca cag gcc ccg cta acc gtg cac gac tcc aaa ctt agc att 432
Met Gln Ser Gln Ala Pro Leu Thr Val His Asp Ser Lys Leu Ser Ile

    130                 135                 140

gcc acc caa gga ccc ctc aca gtg tca gaa gga aag cta gcc ctg caa 480
Ala Thr Gln Gly Pro Leu Thr Val Ser Glu Gly Lys Leu Ala Leu Gln
145                 150                 155                 160

aca tca ggc ccc ctc acc acc acc gat agc agt acc ctt act atc act 528
Thr Ser Gly Pro Leu Thr Thr Thr Asp Ser Ser Thr Leu Thr Ile Thr
                165                 170                 175

gcc tca ccc cct cta act act gcc act ggt agc ttg ggc att gac ttg 576
Ala Ser Pro Pro Leu Thr Thr Ala Thr Gly Ser Leu Gly Ile Asp Leu
            180                 185                 190

aaa gag ccc att tat aca caa aat gga aaa cta gga cta aag tac ggg 624
Lys Glu Pro Ile Tyr Thr Gln Asn Gly Lys Leu Gly Leu Lys Tyr Gly
        195                 200                 205

gct cct ttg cat gta aca gac gac cta aac act ttg acc gta gca act 672
Ala Pro Leu His Val Thr Asp Asp Leu Asn Thr Leu Thr Val Ala Thr
    210                 215                 220

ggt cca ggt gtg act att aat aat act tcc ttg caa act aaa gtt act 720
Gly Pro Gly Val Thr Ile Asn Asn Thr Ser Leu Gln Thr Lys Val Thr
225                 230                 235                 240

gga gcc ttg ggt ttt gat tca caa ggc aat atg caa ctt aat gta gca 768
Gly Ala Leu Gly Phe Asp Ser Gln Gly Asn Met Gln Leu Asn Val Ala
                245                 250                 255

gga gga cta agg att gat tct caa aac aga cgc ctt ata ctt gat gtt 816
Gly Gly Leu Arg Ile Asp Ser Gln Asn Arg Arg Leu Ile Leu Asp Val
            260                 265                 270

agt tat ccg ttt gat gct caa aac caa cta aat cta aga cta gga cag 864
Ser Tyr Pro Phe Asp Ala Gln Asn Gln Leu Asn Leu Arg Leu Gly Gln
        275                 280                 285

ggc cct ctt ttt ata aac tca gcc cac aac ttg gat att aac tac aac 912
Gly Pro Leu Phe Ile Asn Ser Ala His Asn Leu Asp Ile Asn Tyr Asn
    290                 295                 300

aaa ggc ctt tac ttg ttt aca gct tca aac aat tcc aaa aag ctt gag 960
Lys Gly Leu Tyr Leu Phe Thr Ala Ser Asn Asn Ser Lys Lys Leu Glu
305                 310                 315                 320

gtt aac cta agc act gcc aag ggg ttg atg ttt gac gct aca gcc ata 1008
Val Asn Leu Ser Thr Ala Lys Gly Leu Met Phe Asp Ala Thr Ala Ile
                325                 330                 335

gcc att aat gca gga gat ggg ctt gaa ttt ggt tca cct aat gca cca 1056
Ala Ile Asn Ala Gly Asp Gly Leu Glu Phe Gly Ser Pro Asn Ala Pro

            340                 345                 350

aac aca aat ccc ctc aaa aca aaa att ggc cat ggc cta gaa ttt gat 1104
Asn Thr Asn Pro Leu Lys Thr Lys Ile Gly His Gly Leu Glu Phe Asp
        355                 360                 365

tca aac aag gct atg gtt cct aaa cta gga act ggc ctt agt ttt gac 1152
Ser Asn Lys Ala Met Val Pro Lys Leu Gly Thr Gly Leu Ser Phe Asp
    370                 375                 380

agc aca ggt gcc att aca gta gga aac aaa aat aat gat aag cta act 1200
Ser Thr Gly Ala Ile Thr Val Gly Asn Lys Asn Asn Asp Lys Leu Thr
385                 390                 395                 400

ttg tgg acc aca cca gct cca tct cct aac tgt aga cta aat gca gag 1248
Leu Trp Thr Thr Pro Ala Pro Ser Pro Asn Cys Arg Leu Asn Ala Glu
                405                 410                 415

aaa gat gct aaa ctc act ttg gtc tta aca aaa tgt ggc agt caa ata 1296
Lys Asp Ala Lys Leu Thr Leu Val Leu Thr Lys Cys Gly Ser Gln Ile
            420                 425                 430

ctt gct aca gtt tca gtt ttg gct gtt aaa ggc agt ttg gct cca ata 1344
Leu Ala Thr Val Ser Val Leu Ala Val Lys Gly Ser Leu Ala Pro Ile
        435                 440                 445

tct gga aca gtt caa agt gct cat ctt att ata aga ttt gac gaa aat 1392
Ser Gly Thr Val Gln Ser Ala His Leu Ile Ile Arg Phe Asp Glu Asn
    450                 455                 460

gga gtg cta cta aac aat tcc ttc ctg gac cca gaa tat tgg aac ttt 1440
Gly Val Leu Leu Asn Asn Ser Phe Leu Asp Pro Glu Tyr Trp Asn Phe
465                 470                 475                 480

aga aat gga gat ctt act gaa ggc aca gcc tat aca aac gct gtt gga 1488
Arg Asn Gly Asp Leu Thr Glu Gly Thr Ala Tyr Thr Asn Ala Val Gly
                485                 490                 495

ttt atg cct aac cta tca gct tat cca aaa tct cac ggt aaa act gcc 1536
Phe Met Pro Asn Leu Ser Ala Tyr Pro Lys Ser His Gly Lys Thr Ala
            500                 505                 510

aaa agt aac att gtc agt caa gtt tac tta aac gga gac aaa act aaa 1584
Lys Ser Asn Ile Val Ser Gln Val Tyr Leu Asn Gly Asp Lys Thr Lys
        515                 520                 525

cct gta aca cta acc att aca cta aac ggt aca cag gaa aca gga gac 1632
Pro Val Thr Leu Thr Ile Thr Leu Asn Gly Thr Gln Glu Thr Gly Asp
    530                 535                 540

aca act cca agt gca tac tct atg tca ttt tca tgg gac tgg tct ggc 1680
Thr Thr Pro Ser Ala Tyr Ser Met Ser Phe Ser Trp Asp Trp Ser Gly

545                 550                 555                 560

cac aac tac att aat gaa ata ttt gcc acc tcg agt tac act ttt tca     1728
His Asn Tyr Ile Asn Glu Ile Phe Ala Thr Ser Ser Tyr Thr Phe Ser
                565                 570                 575

tac att gcc caa gaa ggt tct gga agt ggt agt ggt tct ggc agc gga     1776
Tyr Ile Ala Gln Glu Gly Ser Gly Ser Gly Ser Gly Ser Gly Ser Gly
            580                 585                 590

tct gga tct aag aag aag aag aag aaa aag aag aaa aag aag aag aag     1824
Ser Gly Ser Lys Lys Lys Lys Lys Lys Lys Lys Lys Lys Lys Lys Lys
        595                 600                 605

aaa aaa aag aag aag aaa aag aaa gga tcc tac tcc atg gag cac ttc     1872
Lys Lys Lys Lys Lys Lys Lys Lys Gly Ser Tyr Ser Met Glu His Phe
    610                 615                 620

cgc tgg ggc aag ccg gtg taa                                         1893
Arg Trp Gly Lys Pro Val
625                 630

<210> 22
<211> 1923
<212> DNA

<213> Artificial Sequence

<220>
<223> DNA coding a modified fiber protein of pWE6.7R-F/asK21MSHb

<220>
<221> CDS
<222> (1)..(1920)

<400> 22
atg aag cgc gca aga ccg tct gaa gat acc ttc aac ccc gtg tat cca     48
Met Lys Arg Ala Arg Pro Ser Glu Asp Thr Phe Asn Pro Val Tyr Pro
1               5                   10                  15

tat gac acg gaa acc ggt cct cca act gtg cct ttt ctt act cct ccc     96
Tyr Asp Thr Glu Thr Gly Pro Pro Thr Val Pro Phe Leu Thr Pro Pro
            20                  25                  30

ttt gta tcc ccc aat ggg ttt caa gag agt ccc cct ggg gta ctc tct     144
Phe Val Ser Pro Asn Gly Phe Gln Glu Ser Pro Pro Gly Val Leu Ser
        35                  40                  45

ttg cgc cta tcc gaa cct cta gtt acc tcc aat ggc atg ctt gcg ctc     192
Leu Arg Leu Ser Glu Pro Leu Val Thr Ser Asn Gly Met Leu Ala Leu
    50                  55                  60

aaa atg ggc aac ggc ctc tct ctg gac gag gcc ggc aac ctt acc tcc 240
Lys Met Gly Asn Gly Leu Ser Leu Asp Glu Ala Gly Asn Leu Thr Ser
65                  70                  75                  80

caa aat gta acc act gtg agc cca cct ctc aaa aaa acc aag tca aac 288
Gln Asn Val Thr Thr Val Ser Pro Pro Leu Lys Lys Thr Lys Ser Asn
                85                  90                  95

ata aac ctg gaa ata tct gca ccc ctc aca gtt acc tca gaa gcc cta 336
Ile Asn Leu Glu Ile Ser Ala Pro Leu Thr Val Thr Ser Glu Ala Leu
            100                 105                 110

act gtg gct gcc gcc gca cct cta atg gtc gcg ggc aac aca ctc acc 384
Thr Val Ala Ala Ala Ala Pro Leu Met Val Ala Gly Asn Thr Leu Thr
        115                 120                 125

atg caa tca cag gcc ccg cta acc gtg cac gac tcc aaa ctt agc att 432
Met Gln Ser Gln Ala Pro Leu Thr Val His Asp Ser Lys Leu Ser Ile
    130                 135                 140

gcc acc caa gga ccc ctc aca gtg tca gaa gga aag cta gcc ctg caa 480
Ala Thr Gln Gly Pro Leu Thr Val Ser Glu Gly Lys Leu Ala Leu Gln
145                 150                 155                 160

aca tca ggc ccc ctc acc acc acc gat agc agt acc ctt act atc act 528
Thr Ser Gly Pro Leu Thr Thr Thr Asp Ser Ser Thr Leu Thr Ile Thr

                165                 170                 175

gcc tca ccc cct cta act act gcc act ggt agc ttg ggc att gac ttg 576
Ala Ser Pro Pro Leu Thr Thr Ala Thr Gly Ser Leu Gly Ile Asp Leu
            180                 185                 190

aaa gag ccc att tat aca caa aat gga aaa cta gga cta aag tac ggg 624
Lys Glu Pro Ile Tyr Thr Gln Asn Gly Lys Leu Gly Leu Lys Tyr Gly
        195                 200                 205

gct cct ttg cat gta aca gac gac cta aac act ttg acc gta gca act 672
Ala Pro Leu His Val Thr Asp Asp Leu Asn Thr Leu Thr Val Ala Thr
    210                 215                 220

ggt cca ggt gtg act att aat aat act tcc ttg caa act aaa gtt act 720
Gly Pro Gly Val Thr Ile Asn Asn Thr Ser Leu Gln Thr Lys Val Thr
225                 230                 235                 240

gga gcc ttg ggt ttt gat tca caa ggc aat atg caa ctt aat gta gca 768
Gly Ala Leu Gly Phe Asp Ser Gln Gly Asn Met Gln Leu Asn Val Ala
                245                 250                 255

gga gga cta agg att gat tct caa aac aga cgc ctt ata ctt gat gtt 816
Gly Gly Leu Arg Ile Asp Ser Gln Asn Arg Arg Leu Ile Leu Asp Val
            260                 265                 270

agt tat ccg ttt gat gct caa aac caa cta aat cta aga cta gga cag 864
Ser Tyr Pro Phe Asp Ala Gln Asn Gln Leu Asn Leu Arg Leu Gly Gln
        275                 280                 285

ggc cct ctt ttt ata aac tca gcc cac aac ttg gat att aac tac aac 912
Gly Pro Leu Phe Ile Asn Ser Ala His Asn Leu Asp Ile Asn Tyr Asn
    290                 295                 300

aaa ggc ctt tac ttg ttt aca gct tca aac aat tcc aaa aag ctt gag 960
Lys Gly Leu Tyr Leu Phe Thr Ala Ser Asn Asn Ser Lys Lys Leu Glu
305                 310                 315                 320

gtt aac cta agc act gcc aag ggg ttg atg ttt gac gct aca gcc ata 1008
Val Asn Leu Ser Thr Ala Lys Gly Leu Met Phe Asp Ala Thr Ala Ile
                325                 330                 335

gcc att aat gca gga gat ggg ctt gaa ttt ggt tca cct aat gca cca 1056
Ala Ile Asn Ala Gly Asp Gly Leu Glu Phe Gly Ser Pro Asn Ala Pro
            340                 345                 350

aac aca aat ccc ctc aaa aca aaa att ggc cat ggc cta gaa ttt gat 1104
Asn Thr Asn Pro Leu Lys Thr Lys Ile Gly His Gly Leu Glu Phe Asp
        355                 360                 365

tca aac aag gct atg gtt cct aaa cta gga act ggc ctt agt ttt gac 1152
Ser Asn Lys Ala Met Val Pro Lys Leu Gly Thr Gly Leu Ser Phe Asp

    370                 375                 380

agc aca ggt gcc att aca gta gga aac aaa aat aat gat aag cta act 1200
Ser Thr Gly Ala Ile Thr Val Gly Asn Lys Asn Asn Asp Lys Leu Thr
385                 390                 395                 400

ttg tgg acc aca cca gct cca tct cct aac tgt aga cta aat gca gag 1248
Leu Trp Thr Thr Pro Ala Pro Ser Pro Asn Cys Arg Leu Asn Ala Glu
                405                 410                 415

aaa gat gct aaa ctc act ttg gtc tta aca aaa tgt ggc agt caa ata 1296
Lys Asp Ala Lys Leu Thr Leu Val Leu Thr Lys Cys Gly Ser Gln Ile
            420                 425                 430

ctt gct aca gtt tca gtt ttg gct gtt aaa ggc agt ttg gct cca ata 1344
Leu Ala Thr Val Ser Val Leu Ala Val Lys Gly Ser Leu Ala Pro Ile
        435                 440                 445

tct gga aca gtt caa agt gct cat ctt att ata aga ttt gac gaa aat 1392
Ser Gly Thr Val Gln Ser Ala His Leu Ile Ile Arg Phe Asp Glu Asn
    450                 455                 460

gga gtg cta cta aac aat tcc ttc ctg gac cca gaa tat tgg aac ttt 1440
Gly Val Leu Leu Asn Asn Ser Phe Leu Asp Pro Glu Tyr Trp Asn Phe
465                 470                 475                 480

aga aat gga gat ctt act gaa ggc aca gcc tat aca aac gct gtt gga 1488
Arg Asn Gly Asp Leu Thr Glu Gly Thr Ala Tyr Thr Asn Ala Val Gly
                485                 490                 495

ttt atg cct aac cta tca gct tat cca aaa tct cac ggt aaa act gcc 1536
Phe Met Pro Asn Leu Ser Ala Tyr Pro Lys Ser His Gly Lys Thr Ala
            500                 505                 510

aaa agt aac att gtc agt caa gtt tac tta aac gga gac aaa act aaa 1584
Lys Ser Asn Ile Val Ser Gln Val Tyr Leu Asn Gly Asp Lys Thr Lys
        515                 520                 525

cct gta aca cta acc att aca cta aac ggt aca cag gaa aca gga gac 1632
Pro Val Thr Leu Thr Ile Thr Leu Asn Gly Thr Gln Glu Thr Gly Asp
    530                 535                 540

aca act cca agt gca tac tct atg tca ttt tca tgg gac tgg tct ggc 1680
Thr Thr Pro Ser Ala Tyr Ser Met Ser Phe Ser Trp Asp Trp Ser Gly
545                 550                 555                 560

cac aac tac att aat gaa ata ttt gcc acc tcg agt tac act ttt tca 1728
His Asn Tyr Ile Asn Glu Ile Phe Ala Thr Ser Ser Tyr Thr Phe Ser
                565                 570                 575

tac att gcc caa gaa cca tca gcc tcc gca tct gct tcc gcc cct gga 1776

Tyr Ile Ala Gln Glu Pro Ser Ala Ser Ala Ser Ala Ser Ala Pro Gly
            580                 585                 590

tct gga tct aag aag aag aag aag aaa aag aag aaa aag aag aag aag 1824
Ser Gly Ser Lys Lys Lys Lys Lys Lys Lys Lys Lys Lys Lys Lys Lys
        595                 600                 605

aaa aaa aag aag aag aaa aag aaa gga tcc gcc gag aag aag gac gag 1872
Lys Lys Lys Lys Lys Lys Lys Lys Gly Ser Ala Glu Lys Lys Asp Glu
    610                 615                 620

ggc ccc tac agg atg gag cac ttc cgc tgg ggc agc ccg ccc aag gac 1920
Gly Pro Tyr Arg Met Glu His Phe Arg Trp Gly Ser Pro Pro Lys Asp
625                 630                 635                 640

taa 1923

<210> 23
<211> 1923
<212> DNA
<213> Artificial Sequence

<220>
<223> DNA coding a modified fiber protein of pWE6.7R-F/gsK21MSHb

<220>

<221> CDS
<222> (1)..(1920)

<400> 23
atg aag cgc gca aga ccg tct gaa gat acc ttc aac ccc gtg tat cca    48
Met Lys Arg Ala Arg Pro Ser Glu Asp Thr Phe Asn Pro Val Tyr Pro
1               5                   10                  15

tat gac acg gaa acc ggt cct cca act gtg cct ttt ctt act cct ccc    96
Tyr Asp Thr Glu Thr Gly Pro Pro Thr Val Pro Phe Leu Thr Pro Pro
            20                  25                  30

ttt gta tcc ccc aat ggg ttt caa gag agt ccc cct ggg gta ctc tct    144
Phe Val Ser Pro Asn Gly Phe Gln Glu Ser Pro Pro Gly Val Leu Ser
        35                  40                  45

ttg cgc cta tcc gaa cct cta gtt acc tcc aat ggc atg ctt gcg ctc    192
Leu Arg Leu Ser Glu Pro Leu Val Thr Ser Asn Gly Met Leu Ala Leu
    50                  55                  60

aaa atg ggc aac ggc ctc tct ctg gac gag gcc ggc aac ctt acc tcc    240
Lys Met Gly Asn Gly Leu Ser Leu Asp Glu Ala Gly Asn Leu Thr Ser
65                  70                  75                  80

caa aat gta acc act gtg agc cca cct ctc aaa aaa acc aag tca aac    288
Gln Asn Val Thr Thr Val Ser Pro Pro Leu Lys Lys Thr Lys Ser Asn

                85                  90                  95

ata aac ctg gaa ata tct gca ccc ctc aca gtt acc tca gaa gcc cta 336
Ile Asn Leu Glu Ile Ser Ala Pro Leu Thr Val Thr Ser Glu Ala Leu
            100                 105                 110

act gtg gct gcc gcc gca cct cta atg gtc gcg ggc aac aca ctc acc 384
Thr Val Ala Ala Ala Ala Pro Leu Met Val Ala Gly Asn Thr Leu Thr
        115                 120                 125

atg caa tca cag gcc ccg cta acc gtg cac gac tcc aaa ctt agc att 432
Met Gln Ser Gln Ala Pro Leu Thr Val His Asp Ser Lys Leu Ser Ile
    130                 135                 140

gcc acc caa gga ccc ctc aca gtg tca gaa gga aag cta gcc ctg caa 480
Ala Thr Gln Gly Pro Leu Thr Val Ser Glu Gly Lys Leu Ala Leu Gln
145                 150                 155                 160

aca tca ggc ccc ctc acc acc acc gat agc agt acc ctt act atc act 528
Thr Ser Gly Pro Leu Thr Thr Thr Asp Ser Ser Thr Leu Thr Ile Thr
                165                 170                 175

gcc tca ccc cct cta act act gcc act ggt agc ttg ggc att gac ttg 576
Ala Ser Pro Pro Leu Thr Thr Ala Thr Gly Ser Leu Gly Ile Asp Leu
            180                 185                 190

aaa gag ccc att tat aca caa aat gga aaa cta gga cta aag tac ggg 624
Lys Glu Pro Ile Tyr Thr Gln Asn Gly Lys Leu Gly Leu Lys Tyr Gly
        195                 200                 205

gct cct ttg cat gta aca gac gac cta aac act ttg acc gta gca act 672
Ala Pro Leu His Val Thr Asp Asp Leu Asn Thr Leu Thr Val Ala Thr
    210                 215                 220

ggt cca ggt gtg act att aat aat act tcc ttg caa act aaa gtt act 720
Gly Pro Gly Val Thr Ile Asn Asn Thr Ser Leu Gln Thr Lys Val Thr
225                 230                 235                 240

gga gcc ttg ggt ttt gat tca caa ggc aat atg caa ctt aat gta gca 768
Gly Ala Leu Gly Phe Asp Ser Gln Gly Asn Met Gln Leu Asn Val Ala
                245                 250                 255

gga gga cta agg att gat tct caa aac aga cgc ctt ata ctt gat gtt 816
Gly Gly Leu Arg Ile Asp Ser Gln Asn Arg Arg Leu Ile Leu Asp Val
            260                 265                 270

agt tat ccg ttt gat gct caa aac caa cta aat cta aga cta gga cag 864
Ser Tyr Pro Phe Asp Ala Gln Asn Gln Leu Asn Leu Arg Leu Gly Gln
        275                 280                 285

ggc cct ctt ttt ata aac tca gcc cac aac ttg gat att aac tac aac 912
Gly Pro Leu Phe Ile Asn Ser Ala His Asn Leu Asp Ile Asn Tyr Asn

    290                 295                 300

aaa ggc ctt tac ttg ttt aca gct tca aac aat tcc aaa aag ctt gag 960
Lys Gly Leu Tyr Leu Phe Thr Ala Ser Asn Asn Ser Lys Lys Leu Glu
305                 310                 315                 320

gtt aac cta agc act gcc aag ggg ttg atg ttt gac gct aca gcc ata 1008
Val Asn Leu Ser Thr Ala Lys Gly Leu Met Phe Asp Ala Thr Ala Ile
                325                 330                 335

gcc att aat gca gga gat ggg ctt gaa ttt ggt tca cct aat gca cca 1056
Ala Ile Asn Ala Gly Asp Gly Leu Glu Phe Gly Ser Pro Asn Ala Pro
            340                 345                 350

aac aca aat ccc ctc aaa aca aaa att ggc cat ggc cta gaa ttt gat 1104
Asn Thr Asn Pro Leu Lys Thr Lys Ile Gly His Gly Leu Glu Phe Asp
        355                 360                 365

tca aac aag gct atg gtt cct aaa cta gga act ggc ctt agt ttt gac 1152
Ser Asn Lys Ala Met Val Pro Lys Leu Gly Thr Gly Leu Ser Phe Asp
    370                 375                 380

agc aca ggt gcc att aca gta gga aac aaa aat aat gat aag cta act 1200
Ser Thr Gly Ala Ile Thr Val Gly Asn Lys Asn Asn Asp Lys Leu Thr
385                 390                 395                 400

ttg tgg acc aca cca gct cca tct cct aac tgt aga cta aat gca gag 1248
Leu Trp Thr Thr Pro Ala Pro Ser Pro Asn Cys Arg Leu Asn Ala Glu
                405                 410                 415

aaa gat gct aaa ctc act ttg gtc tta aca aaa tgt ggc agt caa ata 1296
Lys Asp Ala Lys Leu Thr Leu Val Leu Thr Lys Cys Gly Ser Gln Ile
            420                 425                 430

ctt gct aca gtt tca gtt ttg gct gtt aaa ggc agt ttg gct cca ata 1344
Leu Ala Thr Val Ser Val Leu Ala Val Lys Gly Ser Leu Ala Pro Ile
        435                 440                 445

tct gga aca gtt caa agt gct cat ctt att ata aga ttt gac gaa aat 1392
Ser Gly Thr Val Gln Ser Ala His Leu Ile Ile Arg Phe Asp Glu Asn
    450                 455                 460

gga gtg cta cta aac aat tcc ttc ctg gac cca gaa tat tgg aac ttt 1440
Gly Val Leu Leu Asn Asn Ser Phe Leu Asp Pro Glu Tyr Trp Asn Phe
465                 470                 475                 480

aga aat gga gat ctt act gaa ggc aca gcc tat aca aac gct gtt gga 1488
Arg Asn Gly Asp Leu Thr Glu Gly Thr Ala Tyr Thr Asn Ala Val Gly
                485                 490                 495

ttt atg cct aac cta tca gct tat cca aaa tct cac ggt aaa act gcc 1536
Phe Met Pro Asn Leu Ser Ala Tyr Pro Lys Ser His Gly Lys Thr Ala

            500                 505                 510

aaa agt aac att gtc agt caa gtt tac tta aac gga gac aaa act aaa 1584
Lys Ser Asn Ile Val Ser Gln Val Tyr Leu Asn Gly Asp Lys Thr Lys
        515                 520                 525

cct gta aca cta acc att aca cta aac ggt aca cag gaa aca gga gac 1632
Pro Val Thr Leu Thr Ile Thr Leu Asn Gly Thr Gln Glu Thr Gly Asp
    530                 535                 540

aca act cca agt gca tac tct atg tca ttt tca tgg gac tgg tct ggc 1680
Thr Thr Pro Ser Ala Tyr Ser Met Ser Phe Ser Trp Asp Trp Ser Gly
545                 550                 555                 560

cac aac tac att aat gaa ata ttt gcc acc tcg agt tac act ttt tca 1728
His Asn Tyr Ile Asn Glu Ile Phe Ala Thr Ser Ser Tyr Thr Phe Ser
                565                 570                 575

tac att gcc caa gaa ggt tct gga agt ggt agt ggt tct ggc agc gga 1776
Tyr Ile Ala Gln Glu Gly Ser Gly Ser Gly Ser Gly Ser Gly Ser Gly
            580                 585                 590

tct gga tct aag aag aag aag aag aaa aag aag aaa aag aag aag aag 1824
Ser Gly Ser Lys Lys Lys Lys Lys Lys Lys Lys Lys Lys Lys Lys Lys
        595                 600                 605

aaa aaa aag aag aag aaa aag aaa gga tcc gcc gag aag aag gac gag 1872
Lys Lys Lys Lys Lys Lys Lys Lys Gly Ser Ala Glu Lys Lys Asp Glu
    610                 615                 620

ggc ccc tac agg atg gag cac ttc cgc tgg ggc agc ccg ccc aag gac 1920
Gly Pro Tyr Arg Met Glu His Phe Arg Trp Gly Ser Pro Pro Lys Asp
625                 630                 635                 640

taa 1923

<210> 24
<211> 33
<212> DNA
<213> Artificial Sequence

<220>
<223> DNA coding AS linker

<220>
<221> CDS
<222> (1)..(33)

<400> 24
cca tca gcc tcc gca tct gct tcc gcc cct gga 33
Pro Ser Ala Ser Ala Ser Ala Ser Ala Pro Gly
1               5                   10

<210> 25

<211> 11

<212> PRT

<213> Artificial Sequence

<220>

<223> AS linker peptide

<400> 25

Pro Ser Ala Ser Ala Ser Ala Ser Ala Pro Gly

1               5                   10

<210> 26

<211> 33

<212> DNA

<213> Artificial Sequence

<220>

<223> DNA coding GS linker

<220>

<221> CDS

<222> (1)..(33)

<400> 26

ggt tct gga agt ggt agt ggt tct ggc agc gga                                    33

Gly Ser Gly Ser Gly Ser Gly Ser Gly Ser Gly

 1               5                   10

<210> 27

<211> 11

<212> PRT

<213> Artificial Sequence

<220>

<223> GS linker peptide

<400> 27

Gly Ser Gly Ser Gly Ser Gly Ser Gly Ser Gly

 1               5                   10

<210> 28

<211> 108

<212> DNA

<213> Artificial Sequence

<220>

<223> DNA coding asK21 linker

<220>
<221> CDS
<222> (1)..(108)

<400> 28
cca tca gcc tcc gca tct gct tcc gcc cct gga tct gga tct aag aag    48
Pro Ser Ala Ser Ala Ser Ala Ser Ala Pro Gly Ser Gly Ser Lys Lys
1               5                   10                  15

aag aag aag aaa aag aag aaa aag aag aag aag aaa aaa aag aag aag    96
Lys Lys Lys Lys Lys Lys Lys Lys Lys Lys Lys Lys Lys Lys Lys Lys
            20                  25                  30

aaa aag aaa gga                                                    108
Lys Lys Lys Gly
        35

<210> 29
<211> 36
<212> PRT
<213> Artificial Sequence

<220>

<223> asK21 linker peptide

<400> 29

Pro Ser Ala Ser Ala Ser Ala Ser Ala Pro Gly Ser Gly Ser Lys Lys
1               5                   10                  15

Lys Lys Lys Lys Lys Lys Lys Lys Lys Lys Lys Lys Lys Lys Lys Lys
            20                  25                  30

Lys Lys Lys Gly
        35

<210> 30
<211> 108
<212> DNA
<213> Artificial Sequence

<220>
<223> DNA coding gsK21 linker

<220>
<221> CDS
<222> (1)..(108)

<400> 30

ggt tct gga agt ggt agt ggt tct ggc agc gga tct gga tct aag aag 48
Gly Ser Gly Ser Gly Ser Gly Ser Gly Ser Gly Ser Gly Ser Lys Lys
1               5                   10                  15

aag aag aag aaa aag aag aaa aag aag aag aag aaa aaa aag aag aag 96
Lys Lys Lys Lys Lys Lys Lys Lys Lys Lys Lys Lys Lys Lys Lys Lys
            20                  25                  30

aaa aag aaa gga 108
Lys Lys Lys Gly
        35

<210> 31
<211> 36
<212> PRT
<213> Artificial Sequence

<220>
<223> gsK21 linker peptide

<400> 31
Gly Ser Gly Ser Gly Ser Gly Ser Gly Ser Gly Ser Gly Ser Lys Lys
1               5                   10                  15

Lys Lys Lys Lys Lys Lys Lys Lys Lys Lys Lys Lys Lys Lys Lys Lys

            20                  25                  30

Lys Lys Lys Gly

        35

<210> 32

<211> 605

<212> PRT

<213> Artificial Sequence

<220>

<223> a modified fiber protein encoded in pWE6.7R-F/asMSHa

<400> 32

Met Lys Arg Ala Arg Pro Ser Glu Asp Thr Phe Asn Pro Val Tyr Pro

1               5                   10                  15

Tyr Asp Thr Glu Thr Gly Pro Pro Thr Val Pro Phe Leu Thr Pro Pro

            20                  25                  30

Phe Val Ser Pro Asn Gly Phe Gln Glu Ser Pro Pro Gly Val Leu Ser

        35                  40                  45

Leu Arg Leu Ser Glu Pro Leu Val Thr Ser Asn Gly Met Leu Ala Leu

    50                  55                  60

Lys Met Gly Asn Gly Leu Ser Leu Asp Glu Ala Gly Asn Leu Thr Ser
65                  70                  75                  80

Gln Asn Val Thr Thr Val Ser Pro Pro Leu Lys Lys Thr Lys Ser Asn
                85                  90                  95

Ile Asn Leu Glu Ile Ser Ala Pro Leu Thr Val Thr Ser Glu Ala Leu
            100                 105                 110

Thr Val Ala Ala Ala Ala Pro Leu Met Val Ala Gly Asn Thr Leu Thr
        115                 120                 125

Met Gln Ser Gln Ala Pro Leu Thr Val His Asp Ser Lys Leu Ser Ile
    130                 135                 140

Ala Thr Gln Gly Pro Leu Thr Val Ser Glu Gly Lys Leu Ala Leu Gln
145                 150                 155                 160

Thr Ser Gly Pro Leu Thr Thr Thr Asp Ser Ser Thr Leu Thr Ile Thr
                165                 170                 175

Ala Ser Pro Pro Leu Thr Thr Ala Thr Gly Ser Leu Gly Ile Asp Leu
            180                 185                 190

Lys Glu Pro Ile Tyr Thr Gln Asn Gly Lys Leu Gly Leu Lys Tyr Gly
        195                 200                 205

Ala Pro Leu His Val Thr Asp Asp Leu Asn Thr Leu Thr Val Ala Thr
    210                 215                 220
Gly Pro Gly Val Thr Ile Asn Asn Thr Ser Leu Gln Thr Lys Val Thr
225                 230                 235                 240
Gly Ala Leu Gly Phe Asp Ser Gln Gly Asn Met Gln Leu Asn Val Ala
                245                 250                 255
Gly Gly Leu Arg Ile Asp Ser Gln Asn Arg Arg Leu Ile Leu Asp Val
            260                 265                 270
Ser Tyr Pro Phe Asp Ala Gln Asn Gln Leu Asn Leu Arg Leu Gly Gln
        275                 280                 285
Gly Pro Leu Phe Ile Asn Ser Ala His Asn Leu Asp Ile Asn Tyr Asn
    290                 295                 300
Lys Gly Leu Tyr Leu Phe Thr Ala Ser Asn Asn Ser Lys Lys Leu Glu
305                 310                 315                 320
Val Asn Leu Ser Thr Ala Lys Gly Leu Met Phe Asp Ala Thr Ala Ile
                325                 330                 335
Ala Ile Asn Ala Gly Asp Gly Leu Glu Phe Gly Ser Pro Asn Ala Pro

            340                 345                 350

Asn Thr Asn Pro Leu Lys Thr Lys Ile Gly His Gly Leu Glu Phe Asp
        355                 360                 365

Ser Asn Lys Ala Met Val Pro Lys Leu Gly Thr Gly Leu Ser Phe Asp
    370                 375                 380

Ser Thr Gly Ala Ile Thr Val Gly Asn Lys Asn Asn Asp Lys Leu Thr
385                 390                 395                 400

Leu Trp Thr Thr Pro Ala Pro Ser Pro Asn Cys Arg Leu Asn Ala Glu
                405                 410                 415

Lys Asp Ala Lys Leu Thr Leu Val Leu Thr Lys Cys Gly Ser Gln Ile
            420                 425                 430

Leu Ala Thr Val Ser Val Leu Ala Val Lys Gly Ser Leu Ala Pro Ile
        435                 440                 445

Ser Gly Thr Val Gln Ser Ala His Leu Ile Ile Arg Phe Asp Glu Asn
    450                 455                 460

Gly Val Leu Leu Asn Asn Ser Phe Leu Asp Pro Glu Tyr Trp Asn Phe
465                 470                 475                 480

Arg Asn Gly Asp Leu Thr Glu Gly Thr Ala Tyr Thr Asn Ala Val Gly
                485                 490                 495

Phe Met Pro Asn Leu Ser Ala Tyr Pro Lys Ser His Gly Lys Thr Ala
            500                 505                 510

Lys Ser Asn Ile Val Ser Gln Val Tyr Leu Asn Gly Asp Lys Thr Lys
        515                 520                 525

Pro Val Thr Leu Thr Ile Thr Leu Asn Gly Thr Gln Glu Thr Gly Asp
    530                 535                 540

Thr Thr Pro Ser Ala Tyr Ser Met Ser Phe Ser Trp Asp Trp Ser Gly
545                 550                 555                 560

His Asn Tyr Ile Asn Glu Ile Phe Ala Thr Ser Ser Tyr Thr Phe Ser
                565                 570                 575

Tyr Ile Ala Gln Glu Pro Ser Ala Ser Ala Ser Ala Ser Ala Pro Gly
            580                 585                 590

Ser Tyr Ser Met Glu His Phe Arg Trp Gly Lys Pro Val
        595                 600                 605

<210> 33

<211> 615

<212> PRT

<213> Artificial Sequence

<220>

<223> a modified fiber protein encoded in pWE6.7R-F/asMSHb

<400> 33

Met Lys Arg Ala Arg Pro Ser Glu Asp Thr Phe Asn Pro Val Tyr Pro
1               5                   10                  15

Tyr Asp Thr Glu Thr Gly Pro Pro Thr Val Pro Phe Leu Thr Pro Pro
            20                  25                  30

Phe Val Ser Pro Asn Gly Phe Gln Glu Ser Pro Pro Gly Val Leu Ser
        35                  40                  45

Leu Arg Leu Ser Glu Pro Leu Val Thr Ser Asn Gly Met Leu Ala Leu
    50                  55                  60

Lys Met Gly Asn Gly Leu Ser Leu Asp Glu Ala Gly Asn Leu Thr Ser
65                  70                  75                  80

Gln Asn Val Thr Thr Val Ser Pro Pro Leu Lys Lys Thr Lys Ser Asn
                85                  90                  95

Ile Asn Leu Glu Ile Ser Ala Pro Leu Thr Val Thr Ser Glu Ala Leu
            100                 105                 110
Thr Val Ala Ala Ala Ala Pro Leu Met Val Ala Gly Asn Thr Leu Thr
        115                 120                 125
Met Gln Ser Gln Ala Pro Leu Thr Val His Asp Ser Lys Leu Ser Ile
    130                 135                 140
Ala Thr Gln Gly Pro Leu Thr Val Ser Glu Gly Lys Leu Ala Leu Gln
145                 150                 155                 160
Thr Ser Gly Pro Leu Thr Thr Thr Asp Ser Ser Thr Leu Thr Ile Thr
                165                 170                 175
Ala Ser Pro Pro Leu Thr Thr Ala Thr Gly Ser Leu Gly Ile Asp Leu
            180                 185                 190
Lys Glu Pro Ile Tyr Thr Gln Asn Gly Lys Leu Gly Leu Lys Tyr Gly
        195                 200                 205
Ala Pro Leu His Val Thr Asp Asp Leu Asn Thr Leu Thr Val Ala Thr
    210                 215                 220
Gly Pro Gly Val Thr Ile Asn Asn Thr Ser Leu Gln Thr Lys Val Thr
225                 230                 235                 240

Gly Ala Leu Gly Phe Asp Ser Gln Gly Asn Met Gln Leu Asn Val Ala
                245                 250                 255
Gly Gly Leu Arg Ile Asp Ser Gln Asn Arg Arg Leu Ile Leu Asp Val
            260                 265                 270
Ser Tyr Pro Phe Asp Ala Gln Asn Gln Leu Asn Leu Arg Leu Gly Gln
        275                 280                 285
Gly Pro Leu Phe Ile Asn Ser Ala His Asn Leu Asp Ile Asn Tyr Asn
    290                 295                 300
Lys Gly Leu Tyr Leu Phe Thr Ala Ser Asn Asn Ser Lys Lys Leu Glu
305                 310                 315                 320
Val Asn Leu Ser Thr Ala Lys Gly Leu Met Phe Asp Ala Thr Ala Ile
                325                 330                 335
Ala Ile Asn Ala Gly Asp Gly Leu Glu Phe Gly Ser Pro Asn Ala Pro
            340                 345                 350
Asn Thr Asn Pro Leu Lys Thr Lys Ile Gly His Gly Leu Glu Phe Asp
        355                 360                 365
Ser Asn Lys Ala Met Val Pro Lys Leu Gly Thr Gly Leu Ser Phe Asp

370                 375                 380

Ser Thr Gly Ala Ile Thr Val Gly Asn Lys Asn Asn Asp Lys Leu Thr
385                 390                 395                 400

Leu Trp Thr Thr Pro Ala Pro Ser Pro Asn Cys Arg Leu Asn Ala Glu
                405                 410                 415

Lys Asp Ala Lys Leu Thr Leu Val Leu Thr Lys Cys Gly Ser Gln Ile
            420                 425                 430

Leu Ala Thr Val Ser Val Leu Ala Val Lys Gly Ser Leu Ala Pro Ile
        435                 440                 445

Ser Gly Thr Val Gln Ser Ala His Leu Ile Ile Arg Phe Asp Glu Asn
    450                 455                 460

Gly Val Leu Leu Asn Asn Ser Phe Leu Asp Pro Glu Tyr Trp Asn Phe
465                 470                 475                 480

Arg Asn Gly Asp Leu Thr Glu Gly Thr Ala Tyr Thr Asn Ala Val Gly
                485                 490                 495

Phe Met Pro Asn Leu Ser Ala Tyr Pro Lys Ser His Gly Lys Thr Ala
            500                 505                 510

Lys Ser Asn Ile Val Ser Gln Val Tyr Leu Asn Gly Asp Lys Thr Lys
        515                 520                 525

Pro Val Thr Leu Thr Ile Thr Leu Asn Gly Thr Gln Glu Thr Gly Asp
    530                 535                 540

Thr Thr Pro Ser Ala Tyr Ser Met Ser Phe Ser Trp Asp Trp Ser Gly
545                 550                 555                 560

His Asn Tyr Ile Asn Glu Ile Phe Ala Thr Ser Ser Tyr Thr Phe Ser
                565                 570                 575

Tyr Ile Ala Gln Glu Pro Ser Ala Ser Ala Ser Ala Ser Ala Pro Gly
            580                 585                 590

Ser Ala Glu Lys Lys Asp Glu Gly Pro Tyr Arg Met Glu His Phe Arg
        595                 600                 605

Trp Gly Ser Pro Pro Lys Asp
    610                 615

<210> 34
<211> 605
<212> PRT
<213> Artificial Sequence

<220>

<223> a modified fiber protein encoded in pWE6.7R-F/gsMSHa

<400> 34

Met Lys Arg Ala Arg Pro Ser Glu Asp Thr Phe Asn Pro Val Tyr Pro
1               5                   10                  15

Tyr Asp Thr Glu Thr Gly Pro Pro Thr Val Pro Phe Leu Thr Pro Pro
            20                  25                  30

Phe Val Ser Pro Asn Gly Phe Gln Glu Ser Pro Pro Gly Val Leu Ser
        35                  40                  45

Leu Arg Leu Ser Glu Pro Leu Val Thr Ser Asn Gly Met Leu Ala Leu
    50                  55                  60

Lys Met Gly Asn Gly Leu Ser Leu Asp Glu Ala Gly Asn Leu Thr Ser
65                  70                  75                  80

Gln Asn Val Thr Thr Val Ser Pro Pro Leu Lys Lys Thr Lys Ser Asn
                85                  90                  95

Ile Asn Leu Glu Ile Ser Ala Pro Leu Thr Val Thr Ser Glu Ala Leu
            100                 105                 110

Thr Val Ala Ala Ala Ala Pro Leu Met Val Ala Gly Asn Thr Leu Thr
        115                 120                 125

Met Gln Ser Gln Ala Pro Leu Thr Val His Asp Ser Lys Leu Ser Ile
    130                 135                 140

Ala Thr Gln Gly Pro Leu Thr Val Ser Glu Gly Lys Leu Ala Leu Gln
145                 150                 155                 160

Thr Ser Gly Pro Leu Thr Thr Thr Asp Ser Ser Thr Leu Thr Ile Thr
                165                 170                 175

Ala Ser Pro Pro Leu Thr Thr Ala Thr Gly Ser Leu Gly Ile Asp Leu
            180                 185                 190

Lys Glu Pro Ile Tyr Thr Gln Asn Gly Lys Leu Gly Leu Lys Tyr Gly
        195                 200                 205

Ala Pro Leu His Val Thr Asp Asp Leu Asn Thr Leu Thr Val Ala Thr
    210                 215                 220

Gly Pro Gly Val Thr Ile Asn Asn Thr Ser Leu Gln Thr Lys Val Thr
225                 230                 235                 240

Gly Ala Leu Gly Phe Asp Ser Gln Gly Asn Met Gln Leu Asn Val Ala
                245                 250                 255

Gly Gly Leu Arg Ile Asp Ser Gln Asn Arg Arg Leu Ile Leu Asp Val
            260                 265                 270
Ser Tyr Pro Phe Asp Ala Gln Asn Gln Leu Asn Leu Arg Leu Gly Gln
        275                 280                 285
Gly Pro Leu Phe Ile Asn Ser Ala His Asn Leu Asp Ile Asn Tyr Asn
    290                 295                 300
Lys Gly Leu Tyr Leu Phe Thr Ala Ser Asn Asn Ser Lys Lys Leu Glu
305                 310                 315                 320
Val Asn Leu Ser Thr Ala Lys Gly Leu Met Phe Asp Ala Thr Ala Ile
                325                 330                 335
Ala Ile Asn Ala Gly Asp Gly Leu Glu Phe Gly Ser Pro Asn Ala Pro
            340                 345                 350
Asn Thr Asn Pro Leu Lys Thr Lys Ile Gly His Gly Leu Glu Phe Asp
        355                 360                 365
Ser Asn Lys Ala Met Val Pro Lys Leu Gly Thr Gly Leu Ser Phe Asp
    370                 375                 380
Ser Thr Gly Ala Ile Thr Val Gly Asn Lys Asn Asn Asp Lys Leu Thr

385                 390                 395                 400

Leu Trp Thr Thr Pro Ala Pro Ser Pro Asn Cys Arg Leu Asn Ala Glu

                405                 410                 415

Lys Asp Ala Lys Leu Thr Leu Val Leu Thr Lys Cys Gly Ser Gln Ile

            420                 425                 430

Leu Ala Thr Val Ser Val Leu Ala Val Lys Gly Ser Leu Ala Pro Ile

        435                 440                 445

Ser Gly Thr Val Gln Ser Ala His Leu Ile Ile Arg Phe Asp Glu Asn

    450                 455                 460

Gly Val Leu Leu Asn Asn Ser Phe Leu Asp Pro Glu Tyr Trp Asn Phe

465                 470                 475                 480

Arg Asn Gly Asp Leu Thr Glu Gly Thr Ala Tyr Thr Asn Ala Val Gly

                485                 490                 495

Phe Met Pro Asn Leu Ser Ala Tyr Pro Lys Ser His Gly Lys Thr Ala

            500                 505                 510

Lys Ser Asn Ile Val Ser Gln Val Tyr Leu Asn Gly Asp Lys Thr Lys

        515                 520                 525

Pro Val Thr Leu Thr Ile Thr Leu Asn Gly Thr Gln Glu Thr Gly Asp
    530                 535                 540

Thr Thr Pro Ser Ala Tyr Ser Met Ser Phe Ser Trp Asp Trp Ser Gly
545                 550                 555                 560

His Asn Tyr Ile Asn Glu Ile Phe Ala Thr Ser Ser Tyr Thr Phe Ser
                565                 570                 575

Tyr Ile Ala Gln Glu Gly Ser Gly Ser Gly Ser Gly Ser Gly Ser Gly
            580                 585                 590

Ser Tyr Ser Met Glu His Phe Arg Trp Gly Lys Pro Val
        595                 600                 605

<210> 35
<211> 615
<212> PRT
<213> Artificial Sequence

<220>
<223> a modified fiber protein encoded in pWE6.7R-F/gsMSHb

<400> 35
Met Lys Arg Ala Arg Pro Ser Glu Asp Thr Phe Asn Pro Val Tyr Pro

1                   5                   10                  15

Tyr Asp Thr Glu Thr Gly Pro Pro Thr Val Pro Phe Leu Thr Pro Pro

                20                  25                  30

Phe Val Ser Pro Asn Gly Phe Gln Glu Ser Pro Pro Gly Val Leu Ser

            35                  40                  45

Leu Arg Leu Ser Glu Pro Leu Val Thr Ser Asn Gly Met Leu Ala Leu

        50                  55                  60

Lys Met Gly Asn Gly Leu Ser Leu Asp Glu Ala Gly Asn Leu Thr Ser

65                  70                  75                  80

Gln Asn Val Thr Thr Val Ser Pro Pro Leu Lys Lys Thr Lys Ser Asn

                85                  90                  95

Ile Asn Leu Glu Ile Ser Ala Pro Leu Thr Val Thr Ser Glu Ala Leu

                100                 105                 110

Thr Val Ala Ala Ala Ala Pro Leu Met Val Ala Gly Asn Thr Leu Thr

            115                 120                 125

Met Gln Ser Gln Ala Pro Leu Thr Val His Asp Ser Lys Leu Ser Ile

    130                 135                 140

Ala Thr Gln Gly Pro Leu Thr Val Ser Glu Gly Lys Leu Ala Leu Gln
145                 150                 155                 160

Thr Ser Gly Pro Leu Thr Thr Thr Asp Ser Ser Thr Leu Thr Ile Thr
                165                 170                 175

Ala Ser Pro Pro Leu Thr Thr Ala Thr Gly Ser Leu Gly Ile Asp Leu
            180                 185                 190

Lys Glu Pro Ile Tyr Thr Gln Asn Gly Lys Leu Gly Leu Lys Tyr Gly
        195                 200                 205

Ala Pro Leu His Val Thr Asp Asp Leu Asn Thr Leu Thr Val Ala Thr
    210                 215                 220

Gly Pro Gly Val Thr Ile Asn Asn Thr Ser Leu Gln Thr Lys Val Thr
225                 230                 235                 240

Gly Ala Leu Gly Phe Asp Ser Gln Gly Asn Met Gln Leu Asn Val Ala
                245                 250                 255

Gly Gly Leu Arg Ile Asp Ser Gln Asn Arg Arg Leu Ile Leu Asp Val
            260                 265                 270

Ser Tyr Pro Phe Asp Ala Gln Asn Gln Leu Asn Leu Arg Leu Gly Gln
        275                 280                 285

Gly Pro Leu Phe Ile Asn Ser Ala His Asn Leu Asp Ile Asn Tyr Asn
    290                 295                 300
Lys Gly Leu Tyr Leu Phe Thr Ala Ser Asn Asn Ser Lys Lys Leu Glu
305                 310                 315                 320
Val Asn Leu Ser Thr Ala Lys Gly Leu Met Phe Asp Ala Thr Ala Ile
                325                 330                 335
Ala Ile Asn Ala Gly Asp Gly Leu Glu Phe Gly Ser Pro Asn Ala Pro
            340                 345                 350
Asn Thr Asn Pro Leu Lys Thr Lys Ile Gly His Gly Leu Glu Phe Asp
        355                 360                 365
Ser Asn Lys Ala Met Val Pro Lys Leu Gly Thr Gly Leu Ser Phe Asp
    370                 375                 380
Ser Thr Gly Ala Ile Thr Val Gly Asn Lys Asn Asn Asp Lys Leu Thr
385                 390                 395                 400
Leu Trp Thr Thr Pro Ala Pro Ser Pro Asn Cys Arg Leu Asn Ala Glu
                405                 410                 415
Lys Asp Ala Lys Leu Thr Leu Val Leu Thr Lys Cys Gly Ser Gln Ile

            420                 425                 430

Leu Ala Thr Val Ser Val Leu Ala Val Lys Gly Ser Leu Ala Pro Ile

        435                 440                 445

Ser Gly Thr Val Gln Ser Ala His Leu Ile Ile Arg Phe Asp Glu Asn

    450                 455                 460

Gly Val Leu Leu Asn Asn Ser Phe Leu Asp Pro Glu Tyr Trp Asn Phe

465                 470                 475                 480

Arg Asn Gly Asp Leu Thr Glu Gly Thr Ala Tyr Thr Asn Ala Val Gly

                485                 490                 495

Phe Met Pro Asn Leu Ser Ala Tyr Pro Lys Ser His Gly Lys Thr Ala

            500                 505                 510

Lys Ser Asn Ile Val Ser Gln Val Tyr Leu Asn Gly Asp Lys Thr Lys

        515                 520                 525

Pro Val Thr Leu Thr Ile Thr Leu Asn Gly Thr Gln Glu Thr Gly Asp

    530                 535                 540

Thr Thr Pro Ser Ala Tyr Ser Met Ser Phe Ser Trp Asp Trp Ser Gly

545                 550                 555                 560

His Asn Tyr Ile Asn Glu Ile Phe Ala Thr Ser Ser Tyr Thr Phe Ser
                565                 570                 575

Tyr Ile Ala Gln Glu Gly Ser Gly Ser Gly Ser Gly Ser Gly Ser Gly
            580                 585                 590

Ser Ala Glu Lys Lys Asp Glu Gly Pro Tyr Arg Met Glu His Phe Arg
        595                 600                 605

Trp Gly Ser Pro Pro Lys Asp
    610                 615

<210> 36
<211> 630
<212> PRT
<213> Artificial Sequence

<220>
<223> a modified fiber protein encoded in pWE6.7R-F/asK21MSHa

<400> 36
Met Lys Arg Ala Arg Pro Ser Glu Asp Thr Phe Asn Pro Val Tyr Pro
1               5                   10                  15

Tyr Asp Thr Glu Thr Gly Pro Pro Thr Val Pro Phe Leu Thr Pro Pro

            20                  25                  30

Phe Val Ser Pro Asn Gly Phe Gln Glu Ser Pro Pro Gly Val Leu Ser
        35                  40                  45

Leu Arg Leu Ser Glu Pro Leu Val Thr Ser Asn Gly Met Leu Ala Leu
    50                  55                  60

Lys Met Gly Asn Gly Leu Ser Leu Asp Glu Ala Gly Asn Leu Thr Ser
65                  70                  75                  80

Gln Asn Val Thr Thr Val Ser Pro Pro Leu Lys Lys Thr Lys Ser Asn
                85                  90                  95

Ile Asn Leu Glu Ile Ser Ala Pro Leu Thr Val Thr Ser Glu Ala Leu
            100                 105                 110

Thr Val Ala Ala Ala Ala Pro Leu Met Val Ala Gly Asn Thr Leu Thr
        115                 120                 125

Met Gln Ser Gln Ala Pro Leu Thr Val His Asp Ser Lys Leu Ser Ile
    130                 135                 140

Ala Thr Gln Gly Pro Leu Thr Val Ser Glu Gly Lys Leu Ala Leu Gln
145                 150                 155                 160

Thr Ser Gly Pro Leu Thr Thr Thr Asp Ser Ser Thr Leu Thr Ile Thr
                165                 170                 175
Ala Ser Pro Pro Leu Thr Thr Ala Thr Gly Ser Leu Gly Ile Asp Leu
            180                 185                 190
Lys Glu Pro Ile Tyr Thr Gln Asn Gly Lys Leu Gly Leu Lys Tyr Gly
        195                 200                 205
Ala Pro Leu His Val Thr Asp Asp Leu Asn Thr Leu Thr Val Ala Thr
    210                 215                 220
Gly Pro Gly Val Thr Ile Asn Asn Thr Ser Leu Gln Thr Lys Val Thr
225                 230                 235                 240
Gly Ala Leu Gly Phe Asp Ser Gln Gly Asn Met Gln Leu Asn Val Ala
                245                 250                 255
Gly Gly Leu Arg Ile Asp Ser Gln Asn Arg Arg Leu Ile Leu Asp Val
            260                 265                 270
Ser Tyr Pro Phe Asp Ala Gln Asn Gln Leu Asn Leu Arg Leu Gly Gln
        275                 280                 285
Gly Pro Leu Phe Ile Asn Ser Ala His Asn Leu Asp Ile Asn Tyr Asn
    290                 295                 300

Lys Gly Leu Tyr Leu Phe Thr Ala Ser Asn Asn Ser Lys Lys Leu Glu
305                 310                 315                 320
Val Asn Leu Ser Thr Ala Lys Gly Leu Met Phe Asp Ala Thr Ala Ile
                325                 330                 335
Ala Ile Asn Ala Gly Asp Gly Leu Glu Phe Gly Ser Pro Asn Ala Pro
            340                 345                 350
Asn Thr Asn Pro Leu Lys Thr Lys Ile Gly His Gly Leu Glu Phe Asp
        355                 360                 365
Ser Asn Lys Ala Met Val Pro Lys Leu Gly Thr Gly Leu Ser Phe Asp
    370                 375                 380
Ser Thr Gly Ala Ile Thr Val Gly Asn Lys Asn Asn Asp Lys Leu Thr
385                 390                 395                 400
Leu Trp Thr Thr Pro Ala Pro Ser Pro Asn Cys Arg Leu Asn Ala Glu
                405                 410                 415
Lys Asp Ala Lys Leu Thr Leu Val Leu Thr Lys Cys Gly Ser Gln Ile
            420                 425                 430
Leu Ala Thr Val Ser Val Leu Ala Val Lys Gly Ser Leu Ala Pro Ile

        435                 440                 445

Ser Gly Thr Val Gln Ser Ala His Leu Ile Ile Arg Phe Asp Glu Asn
    450                 455                 460

Gly Val Leu Leu Asn Asn Ser Phe Leu Asp Pro Glu Tyr Trp Asn Phe
465                 470                 475                 480

Arg Asn Gly Asp Leu Thr Glu Gly Thr Ala Tyr Thr Asn Ala Val Gly
                485                 490                 495

Phe Met Pro Asn Leu Ser Ala Tyr Pro Lys Ser His Gly Lys Thr Ala
            500                 505                 510

Lys Ser Asn Ile Val Ser Gln Val Tyr Leu Asn Gly Asp Lys Thr Lys
        515                 520                 525

Pro Val Thr Leu Thr Ile Thr Leu Asn Gly Thr Gln Glu Thr Gly Asp
    530                 535                 540

Thr Thr Pro Ser Ala Tyr Ser Met Ser Phe Ser Trp Asp Trp Ser Gly
545                 550                 555                 560

His Asn Tyr Ile Asn Glu Ile Phe Ala Thr Ser Ser Tyr Thr Phe Ser
                565                 570                 575

Tyr Ile Ala Gln Glu Pro Ser Ala Ser Ala Ser Ala Ser Ala Pro Gly
            580                 585                 590

Ser Gly Ser Lys Lys Lys Lys Lys Lys Lys Lys Lys Lys Lys Lys Lys
        595                 600                 605

Lys Lys Lys Lys Lys Lys Lys Lys Gly Ser Tyr Ser Met Glu His Phe
    610                 615                 620

Arg Trp Gly Lys Pro Val
625                 630

<210> 37
<211> 630
<212> PRT
<213> Artificial Sequence

<220>
<223> a modified fiber protein encoded in pWE6.7R-F/gsK21MSHa

<400> 37
Met Lys Arg Ala Arg Pro Ser Glu Asp Thr Phe Asn Pro Val Tyr Pro
1               5                   10                  15

Tyr Asp Thr Glu Thr Gly Pro Pro Thr Val Pro Phe Leu Thr Pro Pro

            20                  25                  30

Phe Val Ser Pro Asn Gly Phe Gln Glu Ser Pro Pro Gly Val Leu Ser

        35                  40                  45

Leu Arg Leu Ser Glu Pro Leu Val Thr Ser Asn Gly Met Leu Ala Leu

    50                  55                  60

Lys Met Gly Asn Gly Leu Ser Leu Asp Glu Ala Gly Asn Leu Thr Ser

65                  70                  75                  80

Gln Asn Val Thr Thr Val Ser Pro Pro Leu Lys Lys Thr Lys Ser Asn

                85                  90                  95

Ile Asn Leu Glu Ile Ser Ala Pro Leu Thr Val Thr Ser Glu Ala Leu

            100                 105                 110

Thr Val Ala Ala Ala Ala Pro Leu Met Val Ala Gly Asn Thr Leu Thr

        115                 120                 125

Met Gln Ser Gln Ala Pro Leu Thr Val His Asp Ser Lys Leu Ser Ile

    130                 135                 140

Ala Thr Gln Gly Pro Leu Thr Val Ser Glu Gly Lys Leu Ala Leu Gln

145                 150                 155                 160

Thr Ser Gly Pro Leu Thr Thr Thr Asp Ser Ser Thr Leu Thr Ile Thr
                165                 170                 175
Ala Ser Pro Pro Leu Thr Thr Ala Thr Gly Ser Leu Gly Ile Asp Leu
            180                 185                 190
Lys Glu Pro Ile Tyr Thr Gln Asn Gly Lys Leu Gly Leu Lys Tyr Gly
        195                 200                 205
Ala Pro Leu His Val Thr Asp Asp Leu Asn Thr Leu Thr Val Ala Thr
    210                 215                 220
Gly Pro Gly Val Thr Ile Asn Asn Thr Ser Leu Gln Thr Lys Val Thr
225                 230                 235                 240
Gly Ala Leu Gly Phe Asp Ser Gln Gly Asn Met Gln Leu Asn Val Ala
                245                 250                 255
Gly Gly Leu Arg Ile Asp Ser Gln Asn Arg Arg Leu Ile Leu Asp Val
            260                 265                 270
Ser Tyr Pro Phe Asp Ala Gln Asn Gln Leu Asn Leu Arg Leu Gly Gln
        275                 280                 285
Gly Pro Leu Phe Ile Asn Ser Ala His Asn Leu Asp Ile Asn Tyr Asn
    290                 295                 300

Lys Gly Leu Tyr Leu Phe Thr Ala Ser Asn Asn Ser Lys Lys Leu Glu
305                 310                 315                 320
Val Asn Leu Ser Thr Ala Lys Gly Leu Met Phe Asp Ala Thr Ala Ile
                325                 330                 335
Ala Ile Asn Ala Gly Asp Gly Leu Glu Phe Gly Ser Pro Asn Ala Pro
            340                 345                 350
Asn Thr Asn Pro Leu Lys Thr Lys Ile Gly His Gly Leu Glu Phe Asp
        355                 360                 365
Ser Asn Lys Ala Met Val Pro Lys Leu Gly Thr Gly Leu Ser Phe Asp
    370                 375                 380
Ser Thr Gly Ala Ile Thr Val Gly Asn Lys Asn Asn Asp Lys Leu Thr
385                 390                 395                 400
Leu Trp Thr Thr Pro Ala Pro Ser Pro Asn Cys Arg Leu Asn Ala Glu
                405                 410                 415
Lys Asp Ala Lys Leu Thr Leu Val Leu Thr Lys Cys Gly Ser Gln Ile
            420                 425                 430
Leu Ala Thr Val Ser Val Leu Ala Val Lys Gly Ser Leu Ala Pro Ile

        435                 440                 445

Ser Gly Thr Val Gln Ser Ala His Leu Ile Ile Arg Phe Asp Glu Asn
    450                 455                 460

Gly Val Leu Leu Asn Asn Ser Phe Leu Asp Pro Glu Tyr Trp Asn Phe
465                 470                 475                 480

Arg Asn Gly Asp Leu Thr Glu Gly Thr Ala Tyr Thr Asn Ala Val Gly
                485                 490                 495

Phe Met Pro Asn Leu Ser Ala Tyr Pro Lys Ser His Gly Lys Thr Ala
            500                 505                 510

Lys Ser Asn Ile Val Ser Gln Val Tyr Leu Asn Gly Asp Lys Thr Lys
        515                 520                 525

Pro Val Thr Leu Thr Ile Thr Leu Asn Gly Thr Gln Glu Thr Gly Asp
    530                 535                 540

Thr Thr Pro Ser Ala Tyr Ser Met Ser Phe Ser Trp Asp Trp Ser Gly
545                 550                 555                 560

His Asn Tyr Ile Asn Glu Ile Phe Ala Thr Ser Ser Tyr Thr Phe Ser
                565                 570                 575

Tyr Ile Ala Gln Glu Gly Ser Gly Ser Gly Ser Gly Ser Gly Ser Gly
            580                 585                 590

Ser Gly Ser Lys Lys Lys Lys Lys Lys Lys Lys Lys Lys Lys Lys Lys
        595                 600                 605

Lys Lys Lys Lys Lys Lys Lys Lys Gly Ser Tyr Ser Met Glu His Phe
    610                 615                 620

Arg Trp Gly Lys Pro Val
625                 630

<210> 38
<211> 640
<212> PRT
<213> Artificial Sequence

<220>
<223> a modified fiber protein encoded in pWE6.7R-F/asK21MSHb

<400> 38
Met Lys Arg Ala Arg Pro Ser Glu Asp Thr Phe Asn Pro Val Tyr Pro
1               5                   10                  15

Tyr Asp Thr Glu Thr Gly Pro Pro Thr Val Pro Phe Leu Thr Pro Pro

            20                  25                  30

Phe Val Ser Pro Asn Gly Phe Gln Glu Ser Pro Pro Gly Val Leu Ser

        35                  40                  45

Leu Arg Leu Ser Glu Pro Leu Val Thr Ser Asn Gly Met Leu Ala Leu

    50                  55                  60

Lys Met Gly Asn Gly Leu Ser Leu Asp Glu Ala Gly Asn Leu Thr Ser

65                  70                  75                  80

Gln Asn Val Thr Thr Val Ser Pro Pro Leu Lys Lys Thr Lys Ser Asn

                85                  90                  95

Ile Asn Leu Glu Ile Ser Ala Pro Leu Thr Val Thr Ser Glu Ala Leu

            100                 105                 110

Thr Val Ala Ala Ala Ala Pro Leu Met Val Ala Gly Asn Thr Leu Thr

        115                 120                 125

Met Gln Ser Gln Ala Pro Leu Thr Val His Asp Ser Lys Leu Ser Ile

    130                 135                 140

Ala Thr Gln Gly Pro Leu Thr Val Ser Glu Gly Lys Leu Ala Leu Gln

145                 150                 155                 160

Thr Ser Gly Pro Leu Thr Thr Thr Asp Ser Ser Thr Leu Thr Ile Thr
                165                 170                 175

Ala Ser Pro Pro Leu Thr Thr Ala Thr Gly Ser Leu Gly Ile Asp Leu
            180                 185                 190

Lys Glu Pro Ile Tyr Thr Gln Asn Gly Lys Leu Gly Leu Lys Tyr Gly
        195                 200                 205

Ala Pro Leu His Val Thr Asp Asp Leu Asn Thr Leu Thr Val Ala Thr
    210                 215                 220

Gly Pro Gly Val Thr Ile Asn Asn Thr Ser Leu Gln Thr Lys Val Thr
225                 230                 235                 240

Gly Ala Leu Gly Phe Asp Ser Gln Gly Asn Met Gln Leu Asn Val Ala
                245                 250                 255

Gly Gly Leu Arg Ile Asp Ser Gln Asn Arg Arg Leu Ile Leu Asp Val
            260                 265                 270

Ser Tyr Pro Phe Asp Ala Gln Asn Gln Leu Asn Leu Arg Leu Gly Gln
        275                 280                 285

Gly Pro Leu Phe Ile Asn Ser Ala His Asn Leu Asp Ile Asn Tyr Asn
    290                 295                 300

Lys Gly Leu Tyr Leu Phe Thr Ala Ser Asn Asn Ser Lys Lys Leu Glu
305                 310                 315                 320

Val Asn Leu Ser Thr Ala Lys Gly Leu Met Phe Asp Ala Thr Ala Ile
                325                 330                 335

Ala Ile Asn Ala Gly Asp Gly Leu Glu Phe Gly Ser Pro Asn Ala Pro
            340                 345                 350

Asn Thr Asn Pro Leu Lys Thr Lys Ile Gly His Gly Leu Glu Phe Asp
        355                 360                 365

Ser Asn Lys Ala Met Val Pro Lys Leu Gly Thr Gly Leu Ser Phe Asp
    370                 375                 380

Ser Thr Gly Ala Ile Thr Val Gly Asn Lys Asn Asn Asp Lys Leu Thr
385                 390                 395                 400

Leu Trp Thr Thr Pro Ala Pro Ser Pro Asn Cys Arg Leu Asn Ala Glu
                405                 410                 415

Lys Asp Ala Lys Leu Thr Leu Val Leu Thr Lys Cys Gly Ser Gln Ile
            420                 425                 430

Leu Ala Thr Val Ser Val Leu Ala Val Lys Gly Ser Leu Ala Pro Ile

        435                 440                 445

Ser Gly Thr Val Gln Ser Ala His Leu Ile Ile Arg Phe Asp Glu Asn

    450                 455                 460

Gly Val Leu Leu Asn Asn Ser Phe Leu Asp Pro Glu Tyr Trp Asn Phe

465                 470                 475                 480

Arg Asn Gly Asp Leu Thr Glu Gly Thr Ala Tyr Thr Asn Ala Val Gly

                485                 490                 495

Phe Met Pro Asn Leu Ser Ala Tyr Pro Lys Ser His Gly Lys Thr Ala

            500                 505                 510

Lys Ser Asn Ile Val Ser Gln Val Tyr Leu Asn Gly Asp Lys Thr Lys

        515                 520                 525

Pro Val Thr Leu Thr Ile Thr Leu Asn Gly Thr Gln Glu Thr Gly Asp

    530                 535                 540

Thr Thr Pro Ser Ala Tyr Ser Met Ser Phe Ser Trp Asp Trp Ser Gly

545                 550                 555                 560

His Asn Tyr Ile Asn Glu Ile Phe Ala Thr Ser Ser Tyr Thr Phe Ser

                565                 570                 575

Tyr Ile Ala Gln Glu Pro Ser Ala Ser Ala Ser Ala Ser Ala Pro Gly
            580                 585                 590

Ser Gly Ser Lys Lys Lys Lys Lys Lys Lys Lys Lys Lys Lys Lys Lys
        595                 600                 605

Lys Lys Lys Lys Lys Lys Lys Lys Gly Ser Ala Glu Lys Lys Asp Glu
    610                 615                 620

Gly Pro Tyr Arg Met Glu His Phe Arg Trp Gly Ser Pro Pro Lys Asp
625                 630                 635                 640

<210> 39
<211> 640
<212> PRT
<213> Artificial Sequence

<220>
<223> a modified fiber protein encoded in pWE6. 7R-F/gsK21MSHb

<400> 39
Met Lys Arg Ala Arg Pro Ser Glu Asp Thr Phe Asn Pro Val Tyr Pro
1               5                   10                  15

Tyr Asp Thr Glu Thr Gly Pro Pro Thr Val Pro Phe Leu Thr Pro Pro

            20                  25                  30

Phe Val Ser Pro Asn Gly Phe Gln Glu Ser Pro Pro Gly Val Leu Ser

        35                  40                  45

Leu Arg Leu Ser Glu Pro Leu Val Thr Ser Asn Gly Met Leu Ala Leu

    50                  55                  60

Lys Met Gly Asn Gly Leu Ser Leu Asp Glu Ala Gly Asn Leu Thr Ser

65                  70                  75                  80

Gln Asn Val Thr Thr Val Ser Pro Pro Leu Lys Lys Thr Lys Ser Asn

                85                  90                  95

Ile Asn Leu Glu Ile Ser Ala Pro Leu Thr Val Thr Ser Glu Ala Leu

            100                 105                 110

Thr Val Ala Ala Ala Ala Pro Leu Met Val Ala Gly Asn Thr Leu Thr

        115                 120                 125

Met Gln Ser Gln Ala Pro Leu Thr Val His Asp Ser Lys Leu Ser Ile

    130                 135                 140

Ala Thr Gln Gly Pro Leu Thr Val Ser Glu Gly Lys Leu Ala Leu Gln

145                 150                 155                 160

Thr Ser Gly Pro Leu Thr Thr Thr Asp Ser Ser Thr Leu Thr Ile Thr
                165                 170                 175
Ala Ser Pro Pro Leu Thr Thr Ala Thr Gly Ser Leu Gly Ile Asp Leu
            180                 185                 190
Lys Glu Pro Ile Tyr Thr Gln Asn Gly Lys Leu Gly Leu Lys Tyr Gly
        195                 200                 205
Ala Pro Leu His Val Thr Asp Asp Leu Asn Thr Leu Thr Val Ala Thr
    210                 215                 220
Gly Pro Gly Val Thr Ile Asn Asn Thr Ser Leu Gln Thr Lys Val Thr
225                 230                 235                 240
Gly Ala Leu Gly Phe Asp Ser Gln Gly Asn Met Gln Leu Asn Val Ala
                245                 250                 255
Gly Gly Leu Arg Ile Asp Ser Gln Asn Arg Arg Leu Ile Leu Asp Val
            260                 265                 270
Ser Tyr Pro Phe Asp Ala Gln Asn Gln Leu Asn Leu Arg Leu Gly Gln
        275                 280                 285
Gly Pro Leu Phe Ile Asn Ser Ala His Asn Leu Asp Ile Asn Tyr Asn
    290                 295                 300

Lys Gly Leu Tyr Leu Phe Thr Ala Ser Asn Asn Ser Lys Lys Leu Glu
305                 310                 315                 320

Val Asn Leu Ser Thr Ala Lys Gly Leu Met Phe Asp Ala Thr Ala Ile
                325                 330                 335

Ala Ile Asn Ala Gly Asp Gly Leu Glu Phe Gly Ser Pro Asn Ala Pro
            340                 345                 350

Asn Thr Asn Pro Leu Lys Thr Lys Ile Gly His Gly Leu Glu Phe Asp
        355                 360                 365

Ser Asn Lys Ala Met Val Pro Lys Leu Gly Thr Gly Leu Ser Phe Asp
    370                 375                 380

Ser Thr Gly Ala Ile Thr Val Gly Asn Lys Asn Asn Asp Lys Leu Thr
385                 390                 395                 400

Leu Trp Thr Thr Pro Ala Pro Ser Pro Asn Cys Arg Leu Asn Ala Glu
                405                 410                 415

Lys Asp Ala Lys Leu Thr Leu Val Leu Thr Lys Cys Gly Ser Gln Ile
            420                 425                 430

Leu Ala Thr Val Ser Val Leu Ala Val Lys Gly Ser Leu Ala Pro Ile

        435                 440                 445

Ser Gly Thr Val Gln Ser Ala His Leu Ile Ile Arg Phe Asp Glu Asn

    450                 455                 460

Gly Val Leu Leu Asn Asn Ser Phe Leu Asp Pro Glu Tyr Trp Asn Phe

465                 470                 475                 480

Arg Asn Gly Asp Leu Thr Glu Gly Thr Ala Tyr Thr Asn Ala Val Gly

                485                 490                 495

Phe Met Pro Asn Leu Ser Ala Tyr Pro Lys Ser His Gly Lys Thr Ala

            500                 505                 510

Lys Ser Asn Ile Val Ser Gln Val Tyr Leu Asn Gly Asp Lys Thr Lys

        515                 520                 525

Pro Val Thr Leu Thr Ile Thr Leu Asn Gly Thr Gln Glu Thr Gly Asp

    530                 535                 540

Thr Thr Pro Ser Ala Tyr Ser Met Ser Phe Ser Trp Asp Trp Ser Gly

545                 550                 555                 560

His Asn Tyr Ile Asn Glu Ile Phe Ala Thr Ser Ser Tyr Thr Phe Ser

                565                 570                 575

Tyr Ile Ala Gln Glu Gly Ser Gly Ser Gly Ser Gly Ser Gly Ser Gly
            580                 585                 590

Ser Gly Ser Lys Lys Lys Lys Lys Lys Lys Lys Lys Lys Lys Lys Lys
        595                 600                 605

Lys Lys Lys Lys Lys Lys Lys Lys Gly Ser Ala Glu Lys Lys Asp Glu
    610                 615                 620

Gly Pro Tyr Arg Met Glu His Phe Arg Trp Gly Ser Pro Pro Lys Asp
625                 630                 635                 640

## Claims

1. A virus vector comprising a virus structural protein fused with a ligand which specifically binds to a melanocyte-stimulating hormone (MSH) receptor.

2. The virus vector according to claim 1, wherein the virus structural protein is fused with a ligand which specifically binds to the melanocyte-stimulating hormone (MSH) receptor via a linker.

3. The virus vector according to claim 2, wherein the linker is an oligopeptide.

4. The virus vector according to claim 3, wherein the linker has the amino acid sequence represented by any one of SEQ ID NOs:25, 27, 29 and 31.

5. The virus vector according to any one of claims 1 to 4, wherein the virus structural protein is a protein which constructs the outer surface of the virus.

6. The virus vector according to any one of claims 1 to 5, wherein the ligand is a ligand selected from the group consisting of α-MSH, β-MSH, γ-MSH and derivatives of any one thereof.

7. The virus vector according to any one of claim 1 to 6, wherein the virus is selected from viruses belonging to any one of the group consisting of the family *Adenoviridae,* the family *Retroviridae,* the family *Parvoviridae,* the family *Herpesviridae,* the family *Poxviridae,* the family *Papovaviridae*, the family *Hepadnaviridae,* the family *Togaviridae,* the family *Flaviviridae,* the family *Coronaviridae,* the family *Rhabdoviridae,* the family *Paramyxoviridae,* the family *Orthomyxoviridae,* the family *Bunyaviridae,* the family *Arenaviridae* and the family *Reoviridae.*

8. The virus vector according to any one of claims 1 to 6, wherein the virus is a human adenovirus.

9. The virus vector according to any one of claims 1 to 8, wherein the virus contains an exogenous gene.

10. The virus vector according to claim 9, wherein the gene is a gene encoding an enzyme capable of converting a nontoxic prodrug into a drug having a cytotoxicity.

11. The virus vector according to claim 10, wherein the gene is a gene encoding a herpes simplex virus thymine kinase (HSV-tk) or a cytosine deaminase (CD).

12. The virus vector according to claim 9, wherein the gene is a gene encoding a molecule having a cytotoxic activity directly or indirectly.

13. The virus vector according to claim 12, wherein the gene is a gene encoding a cytokine, a cell growth factor or a cell growth inhibiting factor.

14. The virus vector according to claim 12, wherein the gene is a tumor repressor gene, a cell cycle regulator gene or a cell death regulator gene.

15. The virus vector according to claim 9, wherein the exogenous gene is a wild type or mutant gene of adenovirus E1A or E1B or a part of the gene.

16. A medicament comprising the virus vector according to any one of claims 1 to 15.

17. An antitumor agent comprising the virus vector according to any one of claims 1 to 15.

18. The antitumor agent according to 17, wherein the tumor is malignant melanoma.

19. A diagnostic agent of a tumor, comprising the virus vector according to any one of claims 1 to 15.

20. The diagnostic agent according to claim 19, wherein the tumor is malignant melanoma.

21. A linker comprising the amino acid sequence represented by any one of SEQ ID NOs:25, 27, 29 and 31.

22. A DNA encoding the linker according to claim 21.

23. A DNA comprising the nucleotide sequence represented by any one of SEQ ID NOs:24, 26, 28 and 30.

24. A protein comprising the amino acid sequence represented by any one of SEQ ID NOs:32 to 39.

25. A DNA encoding the protein according to claim 24.

26. A DNA comprising the nucleotide sequence represented by any one of SEQ ID NOs:7, 13, 17, 18, 20, 21, 22 and 23.
